# EUROPEAN PATENT SPECIFICATION

(11) **EP 1 554 379 B1**
(45) Date of publication and mention of the grant of the patent: **22.02.2012**
(21) Application number: 03769780.2
(22) Date of filing: 02.10.2003
(51) Int. Cl.: C12N 9/88

(54) **SESQUITERPENE SYNTHASES AND METHODS OF USE**
SESQUITERPENE SYNTHASEN UND VERWERDUNGSMETHODEN
SESQUITERPENE SYNTHASES ET LEURS METHODES D'UTILISATION

(30) Priority: 04.10.2002 US 415765 P; 02.12.2002 WO PCT/IB02/05070
(43) Date of publication of application: 20.07.2005
(60) Divisional application: 10177152.5
(73) Proprietor: Firmenich SA, 1211 Genève 8 (CH)
(72) Inventor: SCHALK, Michel, 74160 Collonges-Sous-Salève (FR); CLARK, Anthony, 75460 Monnetier Mornex (FR)
(74) Representative: Schneiter, Sorin
(86) International application number: PCT/IB2003/005072
(87) International publication number: WO 2004/031376

(56) References cited:
- EP-A- 1 040 765
- DATABASE UniProt 1 June 2002 (2002-06-01), XIA D. & LOUZADA E.: XP002304705 retrieved from EBI Database accession no. Q8RVR2
- DATABASE BIOSIS [Online] BIOSCIENCES INFORMATION SERVICE, PHILADELPHIA, PA, US; June 2001 (2001-06), LOUZADA ELIEZER S ET AL: "Isolation of a terpene synthase gene from mature 'Rio Red' grapefruit using differential display" XP002282507 Database accession no. PREV200100407143 & HORTSCIENCE, vol. 36, no. 3, June 2001 (2001-06), page 440, 98th Annual International Conference of the American Society for Horticultural Science;Sacramento, California, USA; July 21-25, 2001 ISSN: 0018-5345
- MARUYAMA TAKURO ET AL: "Molecular cloning, functional expression and characterization of (E)-beta-farnesene synthase from Citrus junos" BIOLOGICAL AND PHARMACEUTICAL BULLETIN, vol. 24, no. 10, October 2001 (2001-10), pages 1171-1175, XP002282504 ISSN: 0918-6158
- BOHLMANN JORG ET AL: "Plant terpenoid synthases: Molecular biology and phylogenetic analysis" PROCEEDINGS OF THE NATIONAL ACADEMY OF SCIENCES OF USA, NATIONAL ACADEMY OF SCIENCE. WASHINGTON, US, vol. 95, no. 8, 14 April 1998 (1998-04-14), pages 4126-4133, XP002171016 ISSN: 0027-8424
- TRAPP SUSAN C ET AL: "Genomic organization of plant terpene synthases and molecular evolutionary implications" GENETICS, vol. 158, no. 2, June 2001 (2001-06), pages 811-832, XP002282505 ISSN: 0016-6731
- SHARON-ASA LIAT ET AL: "Citrus fruit flavor and aroma biosynthesis: Isolation, functional characterization, and developmental regulation of Cstps1, a key gene in the production of the sesquiterpene aroma compound valencene." PLANT JOURNAL, vol. 36, no. 5, December 2003 (2003-12), pages 664-674, XP002282506 ISSN: 0960-7412 (ISSN print)

## Description

The present invention relates to a method of making valencene using polypeptides and nucleic acids as described herein. It also relates to host cells, plants and microorganism modified to harbor the nucleic acid described herein.

### Background of the Invention

Terpene compounds represent a wide range of natural molecules with a large diversity of structure. The plant kingdom contains the highest diversity of monoterpenes and sesquiterpenes. Often they play a role in defense of the plants against pathogens, insects and herbivores and for attraction of pollinating insect.

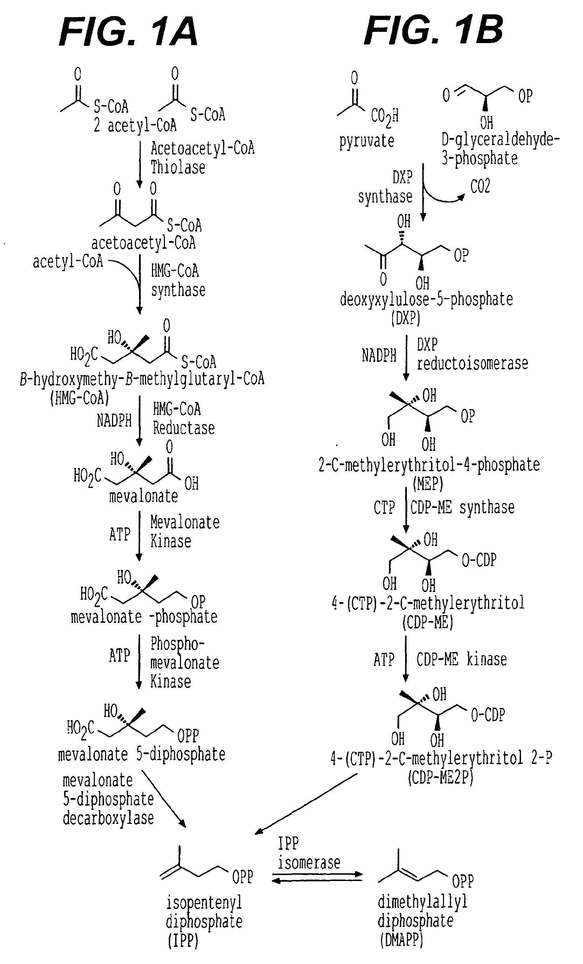

The biosynthesis of terpenes has been extensively studied in many organisms. The common precursor to terpenes is isopentenyl pyrophosphate (IPP) and many of the enzymes catalyzing the steps leading to IPP have been characterized. Two distinct pathways for IPP biosynthesis are currently known (Figure 1). The mevalonate pathway is found in the plants cytosol and in yeast and the non-mevalonate pathway (or deoxyxylulose-5-phosphate (DXP) pathway is found in the plant plastids and in E. coli.

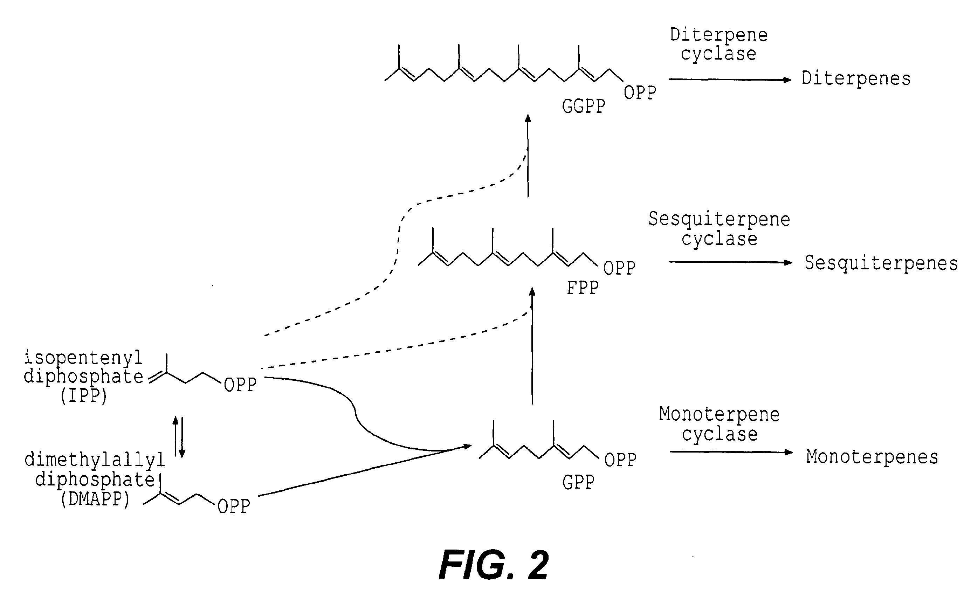

For example, the IPP is isomerized to dimethylallyl diphosphate by the IPP isomerase and these two C5 compounds can be condensed by prenyl transferases to form the acyclic pyrophosphate terpene precursors for each class of terpenes, i.e. geranyl-pyrophosphate (GPP) for the monoterpenes, farnesyl-pyrophosphate (FPP) for the sesquiterpenes, geranylgeranyl-pyrophosphate (GGPP) for the diterpenes (Figure 2). The enzymes catalyzing the cyclisation step of the acyclic precursors are named terpene cyclases or terpene synthases, which are referred to as terpene synthases herein.

These enzymes may be able to catalyze complex multiple step cyclization to form the carbon skeleton of a terpene or sesquiterpene compound. For example, the initial step of the catalyzed cyclisation may be the ionization of the diphosphate group to form an allylic cation. The substrate then undergoes isomerizations and rearrangements which can be controlled by the enzyme active site. The product may, for example, be acyclic, mono-, di or tri-cyclic. A proton may then be released from the carbocation or the carbocation reacts with a water molecule and the terpene hydrocarbon or alcohol is released. Some terpene synthases produce a single product, but many produce multiple products.

A large diversity of terpene structures and sesquiterpene synthases are found in nature. Several sesquiterpene synthase encoding cDNA or genes have been cloned and characterized from different plant sources, e.g, 5-epi-aristolochene synthases form Nicotiana tabacum (Facchini, P.J. and Chappell, J. (1992) Proc. Natl. Acad. Sci. U.S.A. 89, 11088-11092.) and from Capsicum annum (Back, K., et al. (1998) Plant Cell Physiol. 39 (9), 899-904.), a vetispiradiene synthase from Hyoscyamus muticus (Back, K. and Chappell, J. (1995) J. Biol. Chem. 270 (13), 7375-7381.), a (E)-β-farnesene synthase from Mentha piperita (Crock, J., et al. (1997) Proc. Natl. Acad. Sci. U.S.A. 94 (24), 12833-12838.), a δ-selinene synthase and a y-humulene synthase from Abies grandis (Steele, C.L., et al. (1998) J. Biol. Chem. 273 (4), 2078-2089.), δ-cadinene synthases from Gossypium arboreum (Chen, X.Y., et al. (1995) Arch. Biochem. Biophys. 324 (2), 255-266; Chen, X.Y., et al. (1996) J. Nat Prod. 59, 944-951.), a E-α-bisabolene synthase from Abies grandis (Bohlmann, J., et al. (1998) Proc. Natl. Acad. Sci. U.S.A. 95 (12), 6756-6761.), a germacrene C synthase from Lycopersicon esculentum (Colby, S.M., et al. (1998) Proc. Natl. Acad. Sci. U.S.A. 95 (5), 2216-2221.), an epi-cedrol synthase and an amorpha-4,11-diene synthase from Artemisia annua (Mercke, P., et al. (1999) Arch. Biochem. Biophys. 369 (2), 213-222; Mercke, P., et al. (2000) Arch. Biochem. Biophys. 381 (2), 173-180.), and germacrene A synthases from Lactuca sativa, from Cichorium intybus and from Solidago canadensis (Bennett, M.H., et al. (2002) Phytochem. 60, 255-261; Bouwmeester, H.J., et al. (2002) Plant Physiol, 129 (1), 134-144; Prosser I, et al. (2002) Phytochem. 60, 691-702). Uni Prot database discloses a putative terpen synthase from Citrus poradist (Q8RvR2)

Many sesquiterpene compounds are used in perfumery (e.g. patchoulol, nootkatone, santalol, vetivone, sinensal) and many are extracted from plants. As a result, their availability and prices may be subject to fluctuation related to the availability of the plants and the stability of the producing countries. The availability of a plant-independent system for production of sesquiterpenes may therefore be of interest. Due to the structural complexity of some sesquiterpenes, their chemical synthesis at an acceptable cost may not always be feasible.

### Summary of the Invention

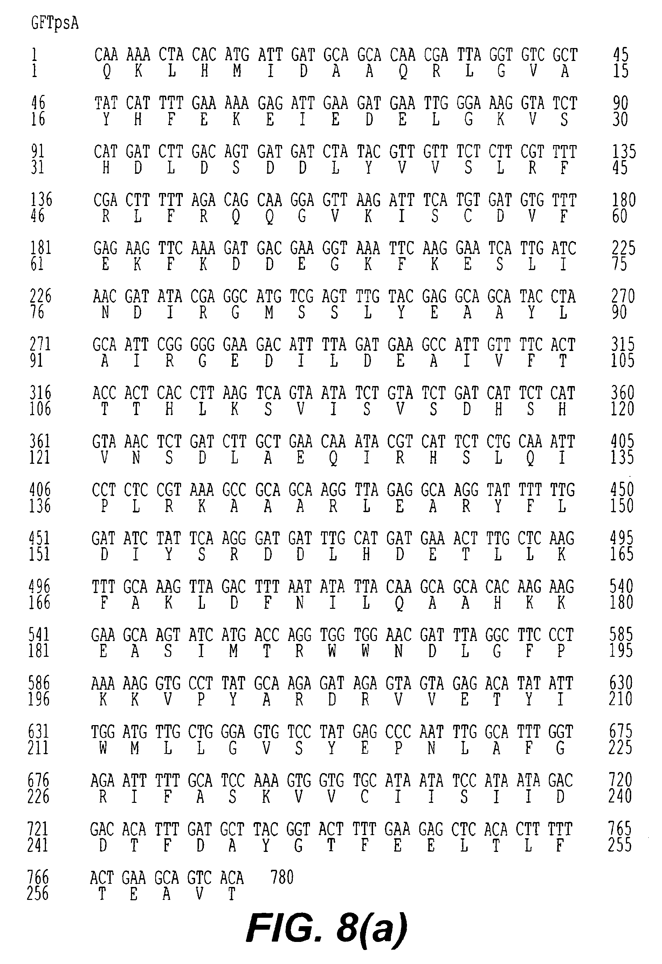

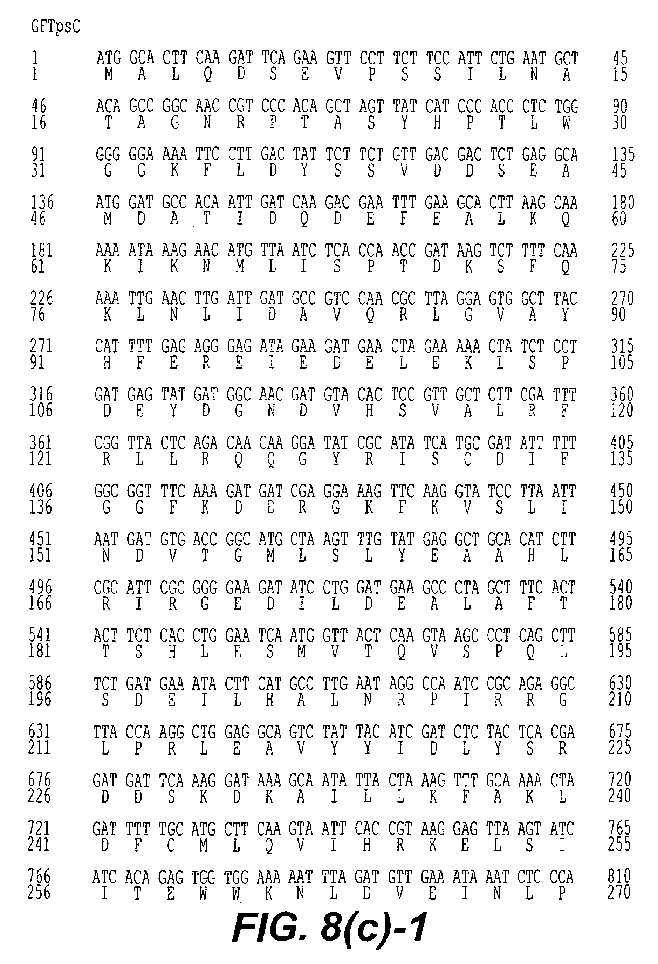

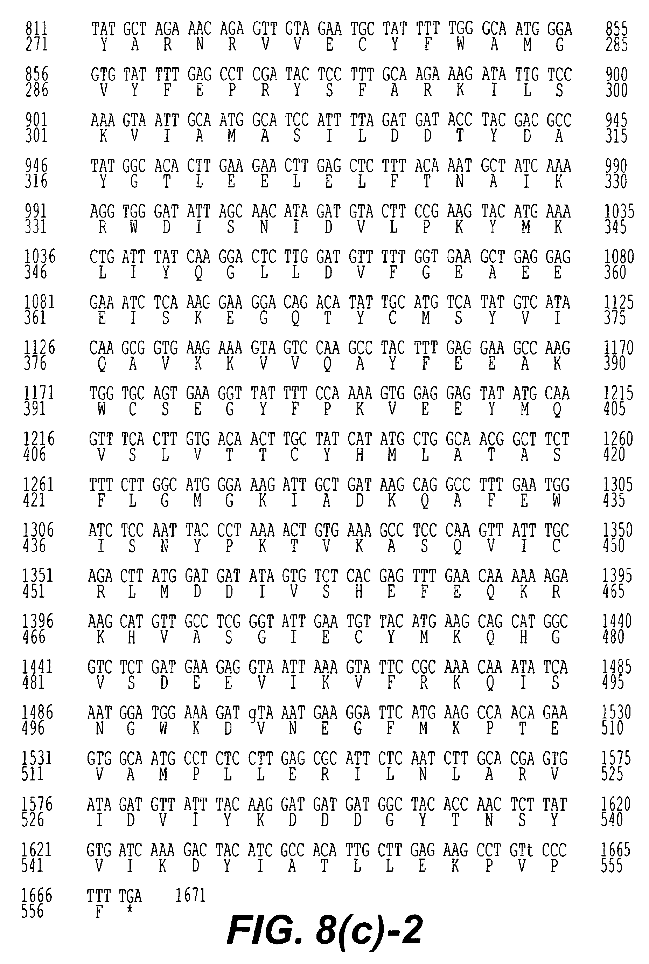

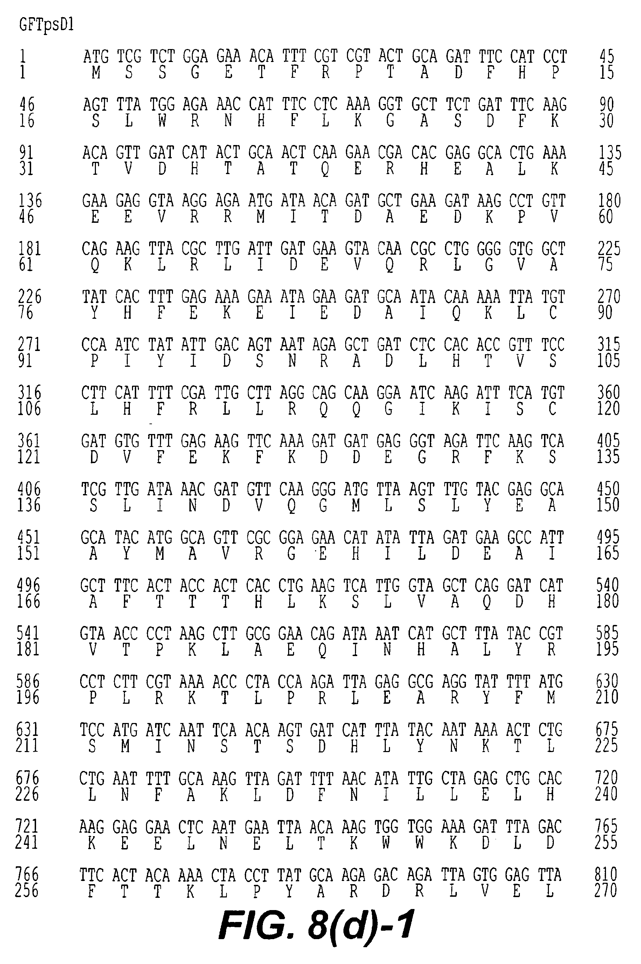

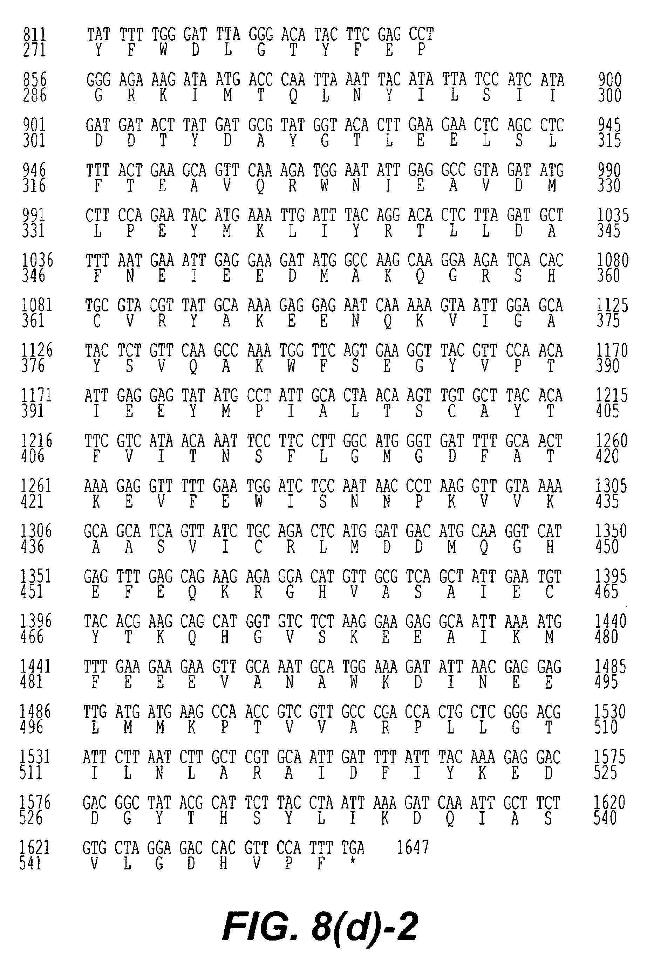

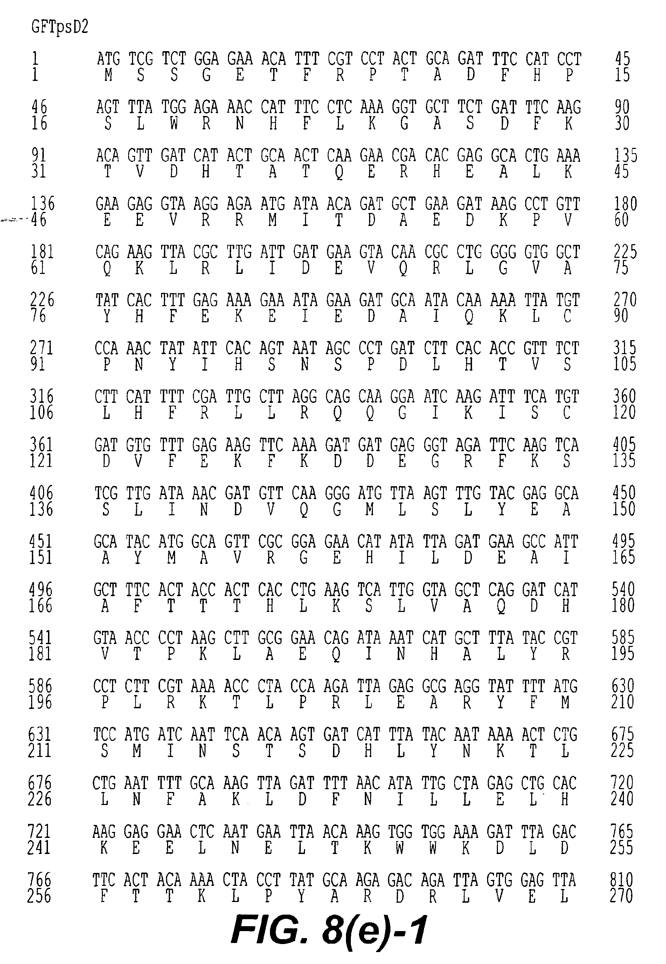

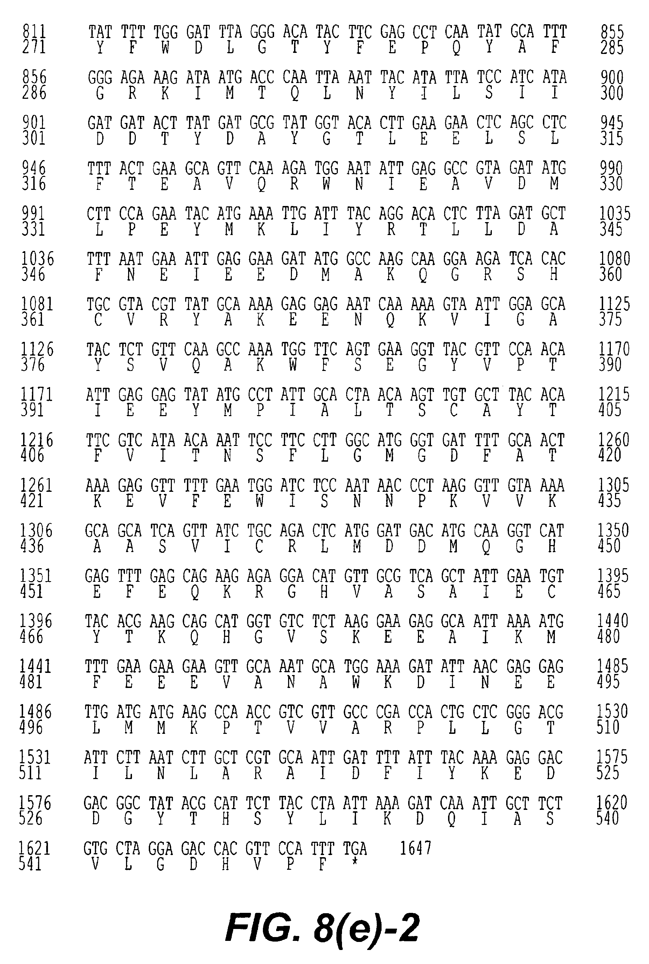

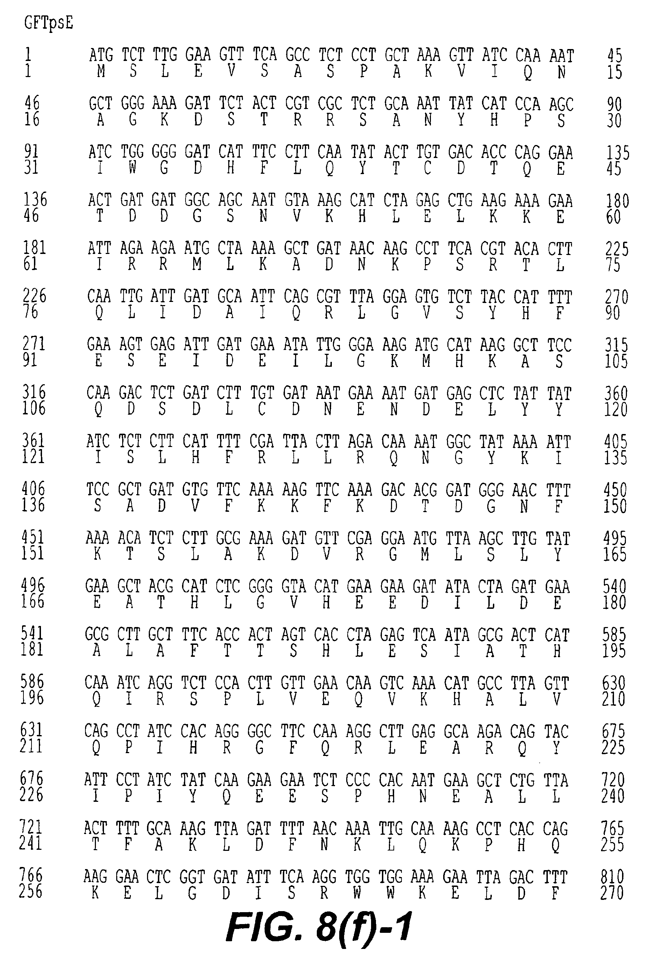

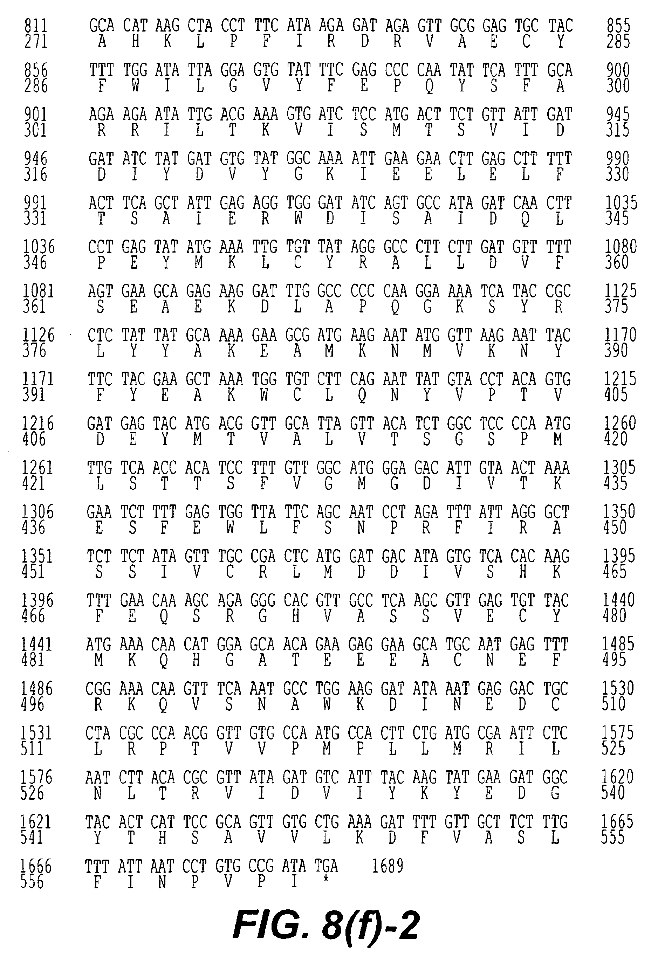

Isolated nucleic acids that encode sesquiterpene synthases are described herein. As used herein, a sesquiterpene synthase may also be referred to by at least one compound produced by the enzyme upon contact with an acyclic pyrophosphate terpene precursor such as farnesyl-pyrophosphate. For example, a sesquiterpene synthase capable of producing valencene as one of its products may be referred to as valencene synthase. Using this convention, examples of nucleic acids include valencene synthase (GFTpsD1 & GFTpsD2) (of Figure 8(d) or 8(e)).

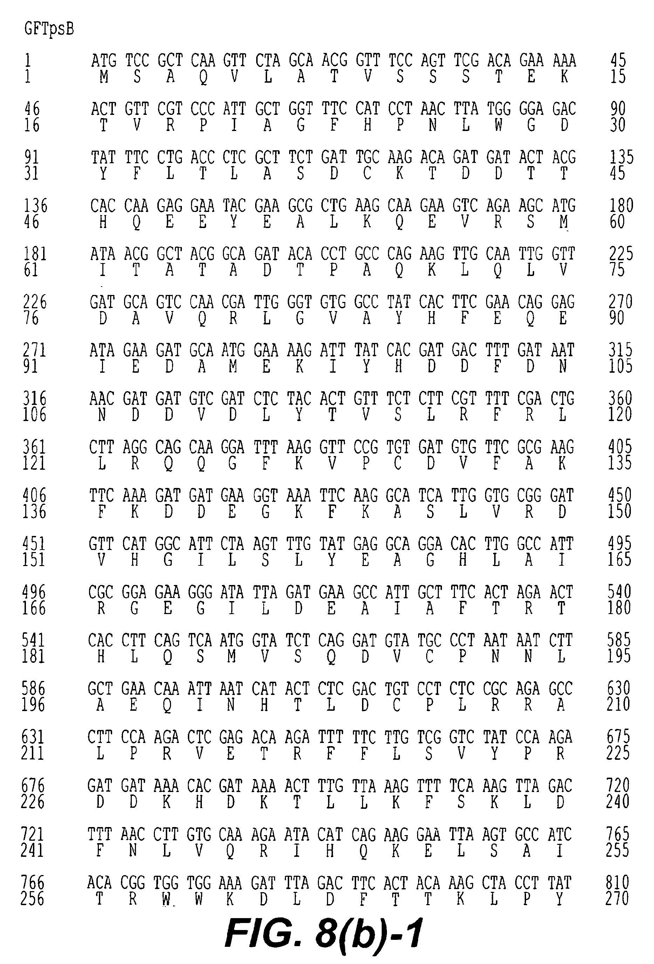

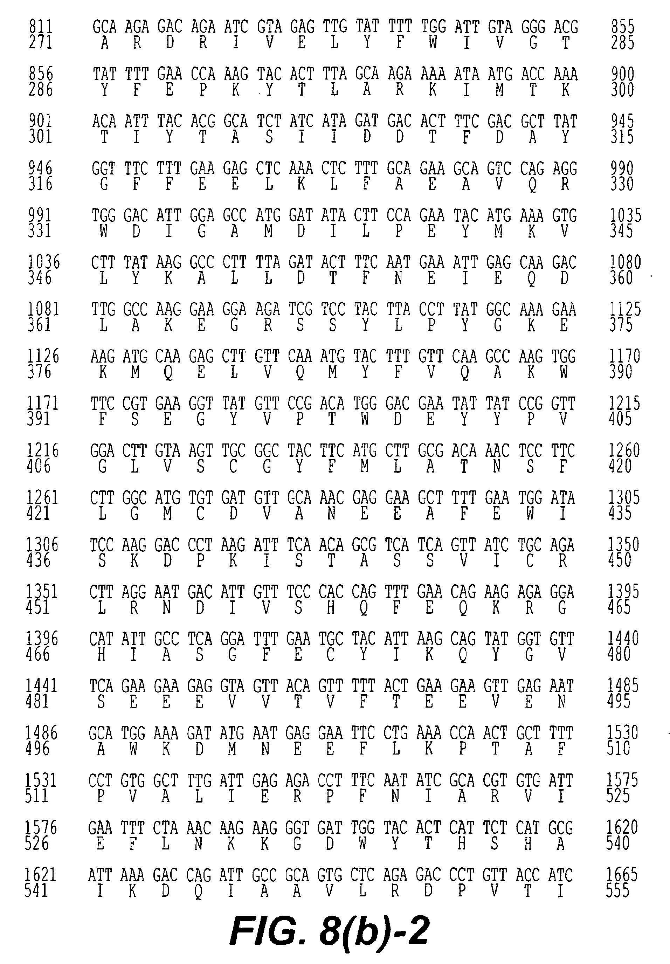

An isolated nucleic acid selected from: (a) a nucleic acid comprising the nucleotide sequence of Figure 8(d) or 8(e); and (b) a nucleic acid encoding the polypeptide of Figure 8(d) or 8(e) is described herein. A polypeptide encoded by a nucleic acid is also described herein. The invention provides a host cell comprising a nucleic acid as described herein; a plant or microorganism modified to harbor a nucleic acid as described herein. Methods of producing a polypeptide comprising culturing host cells of the invention are also described herein.

An isolated polypeptide comprising the amino acid sequence of Figure 8(d) or 8(e) is also described.

A vector comprising at least one nucleic acid chosen from (a) a nucleic acid comprising the nucleotide sequence of Figure 8(d) or 8(e); and (b) a nucleic acid encoding the polypeptide of Figure 8(d) or 8(e) is also described. Methods of making a recombinant host cell comprising introducing a vector described herein into a host cell are also described.

A method of making at least one sesquiterpene synthase comprising culturing a host modified to contain at least one nucleic acid sequence under conditions conducive to the production of said at least one sesquiterpene synthase is described herein. In one embodiment, the at least one nucleic acid is chosen from (a) a nucleic acid comprising the nucleotide sequence of Figure 8(d) or 8(e); and (b) a nucleic acid encoding the polypeptide of Figure 8(d) or 8(e). The host may be chosen from, for example, plants, microorganisms, bacterial cells, yeast cells, plant cells, and animal cells.

In an embodiment the invention provides a method of making valencene comprising 1) contacting fanesyl pyrophosphate with at least one polypeptide encoded by a nucleic acid. In one embodiment, the nucleic acid is chosen from (a) a nucleic acid comprising the nucleotide sequence of Figure 8(d) or 8(e); and (b) a nucleic acid encoding the polypeptide of Figure 8(d) or 8(e); 2) isolating valencene produced in (1).

It is to be understood that both the foregoing general description and the following detailed description are exemplary and explanatory only and are not restrictive of the invention as claimed. Reference will now be made in detail to exemplary embodiments of the present invention.

### Brief Description of the Figures

Figure 1: Example pathways for biosynthesis of isopentenyl pyrophosphate (Mevalonate pathway (A) and deoxyxylulose pathway (B)).

Figure 2: Example terpene biosynthesis from isopentenyl diphosphate.

Figure 3: Structure of example sesquiterpene compounds.

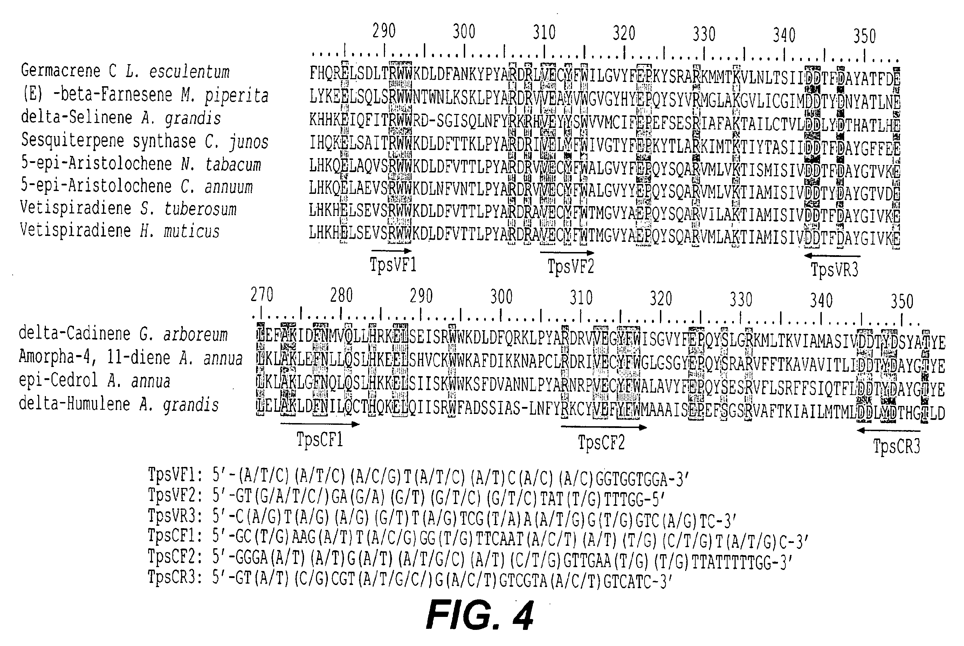

Figure 4: Central part of the alignments of the amino acid sequences of two groups of sesquitepene synthase (Germacrene C L. *esculentum* δ-cadine synthase; (E)-beta-Farnesene *M. piperita;* delta-Selinene *A. grandis;* Sesquiterpene synthase C. junos; 5-epi-Aristolochene *N. tabacum;* 5-epi-Aristolochene C. *annuum;* Vetispiradiene *S.tuberosum;* Vetispiradiene *H. muticus;* delta-Cadinene *G. arboreum;* Amorpha-4,11-diene *A*. *annua;* epi-Cedrol A. *annua;* delta-Humulene *A. grandis* and the sequence of the deduced degenerate primers (TpsVF1; TpsVF2; TpsVR3; TpsCF1; TpsCF2; TpsCR3). The arrows below each alignment show the regions of the alignment used to design the degenerate primers and their orientation.

Figure 5: Alignment of the amino acid sequences deduced from the amplification products obtained by RT-PCR on grapefruit total RNA (GFTpsA; GFTpsB; and GFTpsC).

Figure 6: Amino acid sequence alignment of the sesquiterpene synthases GFTpsA (partial clone), GFTpsB, GFTbsC, GFTpsD1, GFTpsD2, and GFTpsE from C. paradisi.

Figure 7: GC profiles of sesquiterpenes produced by recombinant grapefruit sesquiterpene synthases.

Figure 8: The amino acid and nucleotide sequences of (a) GFTpsA, (b) GFTpsB, (c) GFTbsC, (d) GFTpsD1, (e) GFTpsD2, and (f) GFTpsE.

### Description of the Invention

A terpene is an unsaturated hydrocarbon based on an isoprene unit (C5H8) which may be acyclic or cyclic. Terpene derivatives, include but are not limited to camphor, menthol, terpineol, and borneol, geraniol. Terpenes or Terpenoid, as used herein includes terpenes and terpene derivatives, including compounds that have undergone one or more steps of functionalization such as hydroxylations, isomerizations, oxido-reductions or acylations. As used herein, a sesquiterpene is terpene based on a C15 structure and includes sesquiterpenes and sesquiterpenes derivatives, including compounds that have undergone one or more steps of functionalization such as hydroxylations, isomerizations, oxido-reductions or acylations.

As used herein, a derivative is any compound obtained from a known or hypothetical compound and containing essential elements of the parent substance.

As used herein, sesquiterpene synthase is any enzyme that catalyzes the synthesis of a sesquiterpene.

An isolated nucleic acid selected from: (a) a nucleic acid comprising the nucleotide sequence of Figure 8(d) or 8(e); and (b) a nucleic acid encoding the polypeptide of Figure 8(d) or 8(e) is described herein.

As used herein, one determines whether a polypeptide encoded by a nucleic acid identified herein is a sesquiterpene synthase by the enzyme characterization assay described in the examples herein.

In one embodiment, a nucleic acid and/or polypeptide as described herein is isolated from a citrus, such as, for example a grapefruit or an orange. The isolated nucleotide sequences include those that are substantially free from contaminating endogenous material. The terms "nucleic acid" or "nucleic acid molecule" refer to a deoxyribonucleotide or ribonucleotide polymer in either single-or double-stranded form, and unless otherwise limited, would encompass known analogs of natural nucleotides that can function in a similar manner as naturally occurring nucleotides. A "nucleotide sequence" also refers to a polynucleotide molecule or oligonucleotide molecule in the form of a separate fragment or as a component of a larger nucleic acid. The nucleotide sequence or molecule may also be referred to as a "nucleotide probe." Some of the nucleic acid molecules are derived from DNA or RNA isolated at least once in substantially pure form and in a quantity or concentration enabling identification, manipulation, and recovery of its component nucleotide sequence by standard biochemical methods. Examples of such methods, including methods for PCR protocols that may be used herein, are disclosed in Sambrook et al., Molecular Cloning: A Laboratory Manual, 2nd ed., Cold Spring Harbor Laboratory, Cold Spring Harbor, NY (1989), Current Protocols in Molecular Biology edited by F.A. Ausubel et al., John Wiley and Sons, Inc. (1987), and Innis, M. et al., eds., PCR Protocols: A Guide to Methods and Applications, Academic Press (1990).

As described herein, the nucleic acid molecules described herein include DNA in both single-stranded and double-stranded form, as well as the RNA complement thereof. DNA includes, for example, cDNA, genomic DNA, chemically synthesized DNA, DNA amplified by PCR, and combinations thereof. Genomic DNA, including translated, non-translated and control regions, may be isolated by conventional techniques, e.g., using any one of the cDNAs identified herein, or suitable fragments thereof, as a probe, to identify a piece of genomic DNA which can then be cloned using methods commonly known in the art.

In a further embodiment, the nucleic acid encodes a protein that is a sesquiterpenes synthase, as demonstrated, for example, in the enzyme assay described in the examples. Nucleic acids comprising regions conserved among different species, are also provided.

The isolated nucleic acids described herein may be selected from a nucleic acid encoding the polypeptide of Figure 8(d) or 8(e).

In one embodiment, the polypeptide comprises an amino acid sequence as set out in of Figure 8(d) or 8(e). In one embodiment, the polypeptide described herein is a sesquiterpene synthase, as demonstrated, for example, in the enzyme assay described below.

Due to the degeneracy of the genetic code wherein more than one codon can encode the same amino acid, multiple DNA sequences can code for the same polypeptide. Such variant DNA sequences can result from genetic drift or artificial manipulation (e.g., occurring during PCR amplification or as the product of deliberate mutagenesis of a native sequence).

Deliberate mutagenesis of a native sequence can be carried out using numerous techniques well known in the art. For example, oligonucleotide-directed site-specific mutagenesis procedures can be employed, particularly where it is desired to mutate a gene such that predetermined restriction nucleotides or codons are altered by substitution, deletion or insertion. Exemplary methods of making such alterations are disclosed by Walder et al. (Gene 42:133, 1986); Bauer et al. (Gene 37:73, 1985); Craik (BioTechniques, January 12-19, 1985); Smith et al. (Genetic Engineering: Principles and Methods, Plenum Press, 1981); Kunkel (Proc. Natl. Acad. Sci. USA 82:488, 1985); Kunkel et al. (Methods in Enzymol. 154:367, 1987); and U.S. Patent Nos. 4,518,584 and 4,737,462.

As used herein, the term "polypeptides" refers to a genus of polypeptide or peptide fragments that encompass the amino acid sequences identified herein, as well as smaller fragments. Alternatively, a polypeptide may be defined in terms of its antigenic relatedness to any peptide encoded by the nucleic acid sequences identified herein. Thus, in one embodiment, a polypeptide is defined as an amino acid sequence comprising a linear or 3-dimensional epitope shared with any peptide encoded by the nucleic acid sequences identified herein. Alternatively, a polypeptide is recognized by an antibody that specifically recognizes any peptide encoded by the nucleic acid sequences identified herein. Antibodies are defined to be specifically binding if they bind polypeptides with a Kₐ of greater than or equal to about 10⁷ M⁻¹, such as greater than or equal to 10⁸ M⁻¹.

A polypeptide "variant" as referred to herein means a polypeptide substantially homologous to a native polypeptide, but which has an amino acid sequence different from that encoded by any of the nucleic acid sequences identified herein because of one or more deletions, insertions or substitutions.

Variants can comprise conservatively substituted sequences, meaning that a given amino acid residue is replaced by a residue having similar physiochemical characteristics. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. See Zubay, Biochemistry, Addison-Wesley Pub. Co., (1983). The effects of such substitutions can be calculated using substitution score matrices such a PAM-120, PAM-200, and PAM-250 as discussed in Altschul, (J. Mol. Biol. 219:555-65, 1991). Other such conservative substitutions, for example, substitutions of entire regions having similar hydrophobicity characteristics, are well known.

Variants of the sesquiterpenes synthases described herein may be used to attain desired enhanced or reduced enzymatic activity, modified regiochemistry or stereochemistry, or altered substrate utilization or product distribution. A variant or site direct mutant may be made by any methods known in the art.

As stated above, recombinant and non-recombinant, isolated and purified polypeptides, such as from citrus plants are described herein. Variants and derivatives of native polypeptides can be obtained by isolating naturally-occurring variants, or the nucleotide sequence of variants, of other or same plant lines or species, or by artificially programming mutations of nucleotide sequences coding for native citrus polypeptides. Alterations of the native amino acid sequence can be accomplished by any of a number of conventional methods. Mutations can be introduced at particular loci by synthesizing oligonucleotides containing a mutant sequence, flanked by restriction sites enabling ligation to fragments of the native sequence. Following ligation, the resulting reconstructed sequence encodes an analog having the desired amino acid insertion, substitution, or deletion. Alternatively, oligonucleotide-directed site-specific mutagenesis procedures can be employed to provide an altered gene wherein predetermined codons can be altered by substitution, deletion or insertion.

A vector comprising at least one nucleic acid chosen from (a) a nucleic acid comprising the nucleotide sequence of Figure 8(d) or 8(e); and (b) a nucleic acid encoding the polypeptide of Figure 8(d) or 8(e); can be prepared and used to transform organisms or cells.

A vector as used herein includes any recombinant vector including but not limited to viral vectors, bacteriophages and plasmids.

Recombinant expression vectors containing a nucleic acid sequence identified herein can be prepared using well known methods. In one embodiment, the expression vectors include a cDNA sequence encoding the polypeptide operably linked to suitable transcriptional or translational regulatory nucleotide sequences, such as those derived from a mammalian, microbial, viral, or insect gene. Examples of regulatory sequences include transcriptional promoters, operators, or enhancers, mRNA ribosomal binding sites, and appropriate sequences which control transcription and translation initiation and termination. Nucleotide sequences are "operably linked" when the regulatory sequence functionally relates to the cDNA sequence identified herein. Thus, a promoter nucleotide sequence is operably linked to a cDNA sequence if the promoter nucleotide sequence controls the transcription of the cDNA sequence. The ability to replicate in the desired host cells, usually conferred by an origin of replication, and a selection gene by which transformants are identified can additionally be incorporated into the expression vector.

In addition, sequences encoding appropriate signal peptides that are not naturally associated with the polypeptides identified herein can be incorporated into expression vectors. For example, a DNA sequence for a signal peptide (secretory leader) can be fused in-frame to a nucleotide sequence identified herein so that the polypeptides are initially translated as a fusion protein comprising the signal peptide. A signal peptide that is functional in the intended host cells enhances extracellular secretion of the expressed polypeptide. The signal peptide can be cleaved from the polypeptide upon secretion from the cell.

Fusions of additional peptide sequences at the amino and carboxyl terminal ends of the polypeptides can be used to enhance expression of the polypeptides or aid in the purification of the protein.

In one embodiment, the invention includes a host cell comprising a nucleic acid identified herein. A method of making a recombinant host cell comprising introducing the vectors identified herein, into a host cell is also described. A method of producing a polypeptide comprising culturing the host cells of the invention under conditions to produce the polypeptide is further contemplated. In one embodiment the polypeptide is recovered. Methods of making at least one sesquiterpene synthase comprising culturing a host cell comprising a nucleic acid identified herein, and recovering the sesquiterpene synthase accumulated are also described.

Suitable host cells for expression of polypeptides identified herein include prokaryotes, yeast or higher eukaryotic cells. Appropriate cloning and expression vectors for use with bacterial, fungal, yeast, and mammalian cellular hosts are described, for example, in Pouwels et al., Cloning Vectors: A Laboratory Manual, Elsevier, New York, (1985). Cell-free translation systems could also be employed to produce the disclosed polypeptides using RNAs derived from DNA constructs disclosed herein.

Prokaryotes include gram negative or gram positive organisms, for example, E. coli or Bacilli. Suitable prokaryotic host cells for transformation include, for example, E. coli, Bacillus subtilis, Salmonella typhimurium, and various other species within the genera Pseudomonas, Streptomyces, and Staphylococcus. In a prokaryotic host cell, such as E. coli, the polypeptides can include a N-terminal methionine residue to facilitate expression of the recombinant polypeptide in the prokaryotic host cell. The N-terminal methionine can be cleaved from the expressed recombinant polypeptide.

Examples of useful expression vectors for prokaryotic host cells include those derived from commercially available plasmids such as the cloning vector pET plasmids (Novagen, Madison, WI, USA) or yet pBR322 (ATCC 37017). pBR322 contains genes for ampicillin and tetracycline resistance and thus provides simple means for identifying transformed cells. To construct an expression vector using pBR322, an appropriate promoter and a DNA sequence encoding one or more of the polypeptides identified herein are inserted into the pBR322 vector. Other commercially available vectors include, for example, pKK223-3 (Pharmacia Fine Chemicals, Uppsala, Sweden) and pGEM-1 (Promega Biotec, Madison, WI, USA). Other commercially available vectors include those that are specifically designed for the expression of proteins; these would include pMAL-p2 and pMAL-c2 vectors that are used for the expression of proteins fused to maltose binding protein (New England Biolabs, Beverly, MA, USA).

Promoter sequences commonly used for recombinant prokaryotic host cell expression vectors include bacteriophage T7 promoter (Studier F.W. and Moffatt B.A., J. Mol. Biol. 189:113, 1986), β-lactamase (penicillinase), lactose promoter system (Chang et al., Nature 275:615, 1978; and Goeddel et al., Nature 281:544, 1979), tryptophan (trp) promoter system (Goeddel et al., Nucl. Acids Res. 8:4057, 1980; and EP-A-36776), and tac promoter (Maniatis, MoLecular Cloning: A Laboratory Manual, Cold Spring Harbor Laboratory, p. 412, 1982). A particularly useful prokaryotic host cell expression system employs a phage λ PL promoter and a cl857ts thermolabile repressor sequence. Plasmid vectors available from the American Type Culture Collection ("ATCC"), which incorporate derivatives of the PL promoter, include plasmid pHUB2 (resident in E. coli strain JMB9 (ATCC 37092)) and pPLc28 (resident in E. coli RR1 (ATCC 53082)).

Polypeptides identified herein can also be expressed in yeast host cells, preferably from the Saccharomyces genus (e.g., S. cerevisiae). Other genera of yeast, such as Pichia or Kluyveromyces (e.g. K. lactis), can also be employed. Yeast vectors will often contain an origin of replication sequence from a 2µ yeast plasmid, an autonomously replicating sequence (ARS), a promoter region, sequences for polyadenylation, sequences for transcription termination, and a selectable marker gene. Suitable promoter sequences for yeast vectors include, among others, promoters for metallothionine, 3-phosphoglycerate kinase (Hitzeman et al., J. Biol. Chem. 255:2073, 1980), or other glycolytic enzymes (Hess et al., J. Adv. Enzyme Reg. 7:149, 1968; and Holland et al., Biochem. 17:4900, 1978), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other suitable vectors and promoters for use in yeast expression are further described in Hitzeman, EPA-73,657 or in Fleer et. al., Gene, 107:285-195 (1991); and van den Berg et. al., Bio/Technology, 8:135-139 (1990). Another alternative is the glucose-repressible ADH2 promoter described by Russell et al. (J. Biol. Chem. 258:2674, 1982) and Beier et al. (Nature 300:724, 1982). Shuttle vectors replicable in both yeast and E. coli can be constructed by inserting DNA sequences from pBR322 for selection and replication in E. coli (Ampr gene and origin of replication) into the above-described yeast vectors.

One embodiment of the invention is a plant or a microorganism modified to harbor a nucleic acid identified herein. The plant or microorganism and/or host cell may be modified by any methods known in the art for gene transfer including, for example, the use of deliver devices such as lipids and viral vectors, naked DNA, electroporation and particle-mediated gene transfer.

For example, in one embodiment the invention provides a method of making valencene comprising culturing a host cell modified to contain at least one nucleic acid under conditions conducive to the production of said at least one sesquiterpene synthase wherein said at least one nucleic acid is chosen from (a) a nucleic acid comprising the nucleotide sequence of Figure 8(d) or 8(e); and (b) a nucleic acid encoding the polypeptide of Figure 8(d) or 8(e).

In a further embodiment, the host is bacterial cells, yeast cells, plant cells, and animal cells. As used herein, plant cells and animal cells include the use of plants as a host.

In one embodiment, mammalian or insect host cell culture systems are employed to express recombinant polypeptides identified herein. Baculovirus systems for production of heterologous proteins in insect cells are reviewed by Luckow and Summers, Bio/Technology 6:47 (1988). Established cell lines of mammalian origin also can be employed. Examples of suitable mammalian host cell lines include the COS-7 line of monkey kidney cells (ATCC CRL 1651) (Gluzman et al., Cell 23:175,1981), L cells, C127 cells, 3T3 cells (ATCC CCL 163), Chinese hamster ovary (CHO) cells, HeLa cells, and BHK (ATCC CRL 10) cell lines, and the CV-1/EBNA-1 cell line (ATCC CRL 10478) derived from the African green monkey kidney cell line CVI (ATCC CCL 70) as described by McMahan et al. (EMBO J. 10: 2821,1991).

Established methods for introducing DNA into mammalian cells have been described (Kaufman, R.J., Large Scale Mammalian Cell Culture, 1990, pp. 15-69). Additional protocols using commercially available reagents, such as Lipofectamine (Gibco/BRL) or Lipofectamine-Plus, can be used to transfect cells (Felgner et al., Proc. Natl. Acad. Sci. USA 84:7413-7417, 1987). In addition, electroporation can be used to transfect mammalian cells using conventional procedures, such as those in Sambrook et al. Molecular Cloning: A Laboratory Manual, 2 ed. Vol. 1-3, Cold Spring Harbor Laboratory Press, 1989). Selection of stable transformants can be performed using resistance to cytotoxic drugs as a selection method. Kaufman et al., Meth. in Enzymology 185:487-511, 1990, describes several selection schemes, such as dihydrofolate reductase (DHFR) resistance. A suitable host strain for DHFR selection can be CHO strain DX-B11, which is deficient in DHFR (Urlaub and Chasin, Proc. Natl. Acad. Sci. USA 77:4216-4220, 1980). A plasmid expressing the DHFR cDNA can be introduced into strain DX-B11, and only cells that contain the plasmid can grow in the appropriate selective media.

Transcriptional and translational control sequences for mammalian host cell expression vectors can be excised from viral genomes. Commonly used promoter sequences and enhancer sequences are derived from polyoma virus, adenovirus 2, simian virus 40 (SV40), and human cytomegalovirus. DNA sequences derived from the SV40 viral genome, for example, SV40 origin, early and later promoter, enhancer, splice, and polyadenylation sites can be used to provide other genetic elements for expression of a structural gene sequence in a mammalian host cell. Viral early and late promoters are particularly useful because both are easily obtained from a viral genome as a fragment, which can also contain a viral origin of replication (Fiers et al., Nature 273:113, 1978; Kaufman, Meth. in Enzymology, 1990).

There are several methods known in the art for the creation of transgenic plants. These include, but are not limited to: electroporation of plant protoplasts, liposome-mediated transformation, polyethylene-glycol-mediated transformation, microinjection of plant cells, and transformation using viruses. In one embodiment, direct gene transfer by particle bombardment is utilized.

Direct gene transfer by particle bombardment provides an example for transforming plant tissue. In this technique a particle, or microprojectile, coated with DNA is shot through the physical barriers of the cell. Particle bombardment can be used to introduce DNA into any target tissue that is penetrable by DNA coated particles, but for stable transformation, it is imperative that regenerable cells be used. Typically, the particles are made of gold or tungsten. The particles are coated with DNA using either CaCl2 or ethanol precipitation methods which are commonly known in the art.

DNA coated particles are shot out of a particle gun. A suitable particle gun can be purchased from Bio-Rad Laboratories (Hercules, CA). Particle penetration is controlled by varying parameters such as the intensity of the explosive burst, the size of the particles, or the distance particles must travel to reach the target tissue.

The DNA used for coating the particles may comprise an expression cassette suitable for driving the expression of the gene of interest that will comprise a promoter operably linked to the gene of interest.

Methods for performing direct gene transfer by particle bombardment are disclosed in U.S. Patent 5,990,387 to Tomes et al.

In one embodiment, the cDNAs identified herein may be expressed in such a way as to produce either sense or antisense RNA. Antisense RNA is RNA that has a sequence which is the reverse complement of the mRNA (sense RNA) encoded by a gene. A vector that will drive the expression of antisense RNA is one in which the cDNA is placed in "reverse orientation" with respect to the promoter such that the non-coding strand (rather than the coding strand) is transcribed. The expression of antisense RNA can be used to down-modulate the expression of the protein encoded by the mRNA to which the antisense RNA is complementary. Vectors producing antisense RNA's could be used to make transgenic plants, as described above.

In one embodiment, transfected DNA is integrated into a chromosome of a non-human organism such that a stable recombinant systems results. Any chromosomal integration method known in the art may be used in the practice of the invention, including but not limited to, recombinase-mediated cassette exchange (RMCE), viral site specific chromosomal insertion, adenovirus, and pronuclear injection.

A further embodiment of the invention is methods of making valencene, for example, using the nucleotides and polypeptides described herein. Examples include methods of making valencene comprising contacting farnesyl pyrophosphate with at least one polypeptide encoded by a nucleic acid chosen from (a) a nucleic acid comprising the nucleotide sequence of Figure 8(d) or 8(e); and (b) a nucleic acid encoding the polypeptide of Figure 8(d) or 8(e). Another example is a method of making valencene comprising contacting farnesyl pyrophosphate with at least one polypeptide of Figure 8(d) or 8(e) and isolating the valencene produced.

Valencene ((+)-(1R)-1,2,3,5,6,7,8,8A-octahydro-7-isopropenyl-1α,8Aα-dimethylnaphthalene), is represented in Figure 2. Valencene may be isolated by any method used in the art including but not limited to chromatography, extraction and distillation.

In one embodiment, the distribution of products or the actual products formed may be altered by varying the pH at which the synthase contacts the farnesyl-pyrophosphate. In one embodiment, the pH is 7. In a further embodiment the pH is less than 7, such as, for example, 6, 5, 4, and 3.

Also within the practice of the invention is a microorganism or plant that is used to construct a platform for high level production of a substrate of sesquiterpene synthases (e.g., FPP) and the introduction of a nucleic acid described herein into the plant or microorganism. For example, at least one nucleic acid described herein that encodes a sesquiterpene synthase is incorporated into a microorganism or plant that produces FPP thereby effecting conversion of FPP to valencene, and the subsequent metabolic production of valencene. In one embodiment, this results in a platform for the high level production of valencene.

The nucleic acids described herein also provide for a method of identifying a sesquiterpene synthases comprising constructing a DNA library using the nucleic acids described herein, screening the library for nucleic acids which encode for at least one sesquiterpene synthase. The DNA library using the nucleic acids described herein may be constructed by any process known in the art where DNA sequences are created using the nucleic acids described herein as a starting point, including but not limited to DNA suffling. In such a method, the library may be screened for sesquiterpene synthases using a functional assay to find a target nucleic acid that encodes a sesquiterpene synthase. The activity of a sesquiterpene synthase may be analyzed using, for example, the methods described herein. In one embodiment, high through put screening is utilized to analyze the activity of the encoded polypeptides.

As used herein a "nucleotide probe" is defined as an oligonucleotide or polynucleotide capable of binding to a target nucleic acid of complementary sequence through one or more types of chemical bonds, through complementary base pairing, or through hydrogen bond formation. As described above, the oligonucleotide probe may include natural (ie. A, G, C, or T) or modified bases (7-deazaguanosine, inosine, etc.). In addition, bases in a nucleotide probe may be joined by a linkage other than a phosphodiester bond, so long as it does not prevent hybridization. Thus, oligonucleotide probes may have constituent bases joined by peptide bonds rather than phosphodiester linkages.

A "target nucleic acid" herein refers to a nucleic acid to which the nucleotide probe or molecule can specifically hybridize. The probe is designed to determine the presence or absence of the target nucleic acid, and the amount of target nucleic acid. The target nucleic acid has a sequence that is complementary to the nucleic acid sequence of the corresponding probe directed to the target. As recognized by one of skill in the art, the probe may also contain additional nucleic acids or other moieties, such as labels, which may not specifically hybridize to the target. The term target nucleic acid may refer to the specific nucleotide sequence of a larger nucleic acid to which the probe is directed or to the overall sequence (e.g., gene or mRNA). One skilled in the art will recognize the full utility under various conditions.

Other than in the operating example, or where otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the specification and claims are approximations that may vary depending upon the desired properties sought to be obtained by the present invention. At the very least, and not as an attempt to limit the application of the doctrine of equivalents to the scope of the claims, each numerical parameter should be construed in light of the number of significant digits and ordinary rounding approaches.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements. The following examples are intended to illustrate the invention without limiting the scope as a result. The percentages are given on a weight basis.

The following examples are intended to illustrate the invention without limiting the scope as a result.

### Examples

### Material

A grapefruit (Citrus paradisi) flavedo was used as starting material for the experiments described herein. The flavedo (external, colored portion of the fruit peel) contains the oil glands that are the site of terpene biosynthesis. The grapefruit flavedo was prepared at Simone Gatto (Sicily) from freshly picked ripening fruits. The flavedo (3-4 mm thickness) was cutoff, immediately frozen and kept frozen during the transport and all subsequent steps in order to prevent from degradation.

### Example 1: Isolating Sesquiterpene Synthase cDNA using RT-PCR

The deduced amino-acid sequences of plant sesquiterpene synthases were aligned to identify conserved regions and design plant sesquiterpene synthases-specific oligonucleotides. In order to obtain better sequence homology, the sequences were separated into two groups (Figure 4). The first group contained the sequences of the Germacrene C synthase from Lycopersicon esculentum cv. VFNT cherry (Colby et al, 1998), the (E)-β-farnesene synthase from Mentha x piperita (Crock et al, 1997), the δ-selinene synthase from Abies grandis (Steele et al, 1998), a sesquiterpene synthase from Citrus junos (GenBank accession no. AF288465) the 5-epi aristolochene synthases from Nicotiana tabacum (Facchini and Chappell, 1992) and from Capsicum annuum (Back et al, 1998), the vetispiradiene synthases from Solanum tuberosum and from Hyoscyamus muticus (Back and Chappel, 1995). The second group contained sequences of the (+)-δ-cadinene synthases from Gossypium arboreum (Chen et al. 1995), the amorpha-4,11-diene synthase (Mercke et al, 2000) and the epi-cedrol synthase (Merck et al, 1999) from Artemisia annua and the γ-humulene synthase from Abies grandis (Steele et al, 1998). The highest sequence homology was found in the central part of the sequences. Three regions containing sufficiently conserved amino-acids were selected and degenerated oligonucleotides specific for these regions were designed (i.e. two forward and one reverse primers were deduced from each alignment) (Figure 4).

RT-PCR was performed using total RNA from grapefruit flavedo prepared by the Hot Borate technique and the different combination of the forward and reverse degenerated primers. The Hot Borate method for total RNA extraction was adapted from Wan and Wilkins (Wan et al. (1994) Anal. Biochem. 223, 7-12.). Tissues were crushed to a fine powder in liquid nitrogen using a mortar and pestle. The powder was added to 5 ml extraction buffer (200 mM borate, 30 mM EGTA, 10 mM DTT, 1% SDS, 1% NA deoxycholate, 2% PVP, 0.5% nonidet NP-40) preheated at 80°C. The mixture was homogenized using an UltraturaxTM homogenizer and filtered through two layer of MiraclothTM filter (Calbiochem). The mixture was incubated 90 minutes at 42°C in the presence of 0.5 mg/ml proteinase K (for protein/ribonuclease digestion). KCI was then added to 1 M final concentration and, after 1 hour incubation on ice, the mixture was placed in a centrifuge for 10 min at 10000g and 4°C. The supernatant was recovered and 1/3 volume of 8M LiCl was added. The mixture was incubated over night at 4°C and placed in a centrifuge for 20 minutes at 10000g and 4°C. The supernatant was discarded and, the pellet washed with 2M LiCl and centrifuged again as before. The pellet was then resuspended in RNase-free distillated water, and 0.15 vol of 2 M potassium acetate solution and 2.5 volumes absolute ethanol were added. The RNA were precipitated by incubating 2 hours at -20°C and pelleted by centrifugation as before. The RNA pellet was washed with 80% ethanol and resuspended in RNase free distillated water.

The concentration of RNA was estimated from the OD at 260 nm. The integrity of the RNA was evaluated on an agarose gel by verifying the integrity of the ribosomic RNA bands.

RT-PCR was performed using the Qiagen OneStep RT-PCR Kit and an Eppendorf Mastercycler gradiant thermal cycler. Typical reaction mixtures contained 10 µl 5X Qiagen OneStep RT-PCR buffer, 400 µM each dNTP, 400 nM each primer, 2 µl Qiagen OneStep RT-PCR Enzyme Mix, 1 µl RNasin® Ribonuclease Inhibitor (Promega Co.) and 1 µg total RNA in a final volume of 50 µl. The thermal cycler conditions were: 30 min at 50°C (reverse transcription); 15 min at 95°C (DNA polymerase activation); 40 cycles of 45 sec at 94°C, 10 sec at 42°C to 45°C (depending on the primer used), 45 sec to 90 sec (depending on the size of the DNA fragment to be amplified) at 72°C; and 10 min at 72°C.

The size of the PCR products was evaluated on a 1 % agarose gel. The bands corresponding to the expected size were excised from the gel, purified using the QIAquick® Gel Extraction Kit (Qiagen) and cloned in the pCR®2.1-TOPO vector using the TOPO TA cloning Kit (Invitrogen). Inserted cDNAs were then subject to DNA sequencing and the sequence compared against the GenBank non redundant protein database (NCBI) using the BLASTX algorithm (Altschul et al 1990).

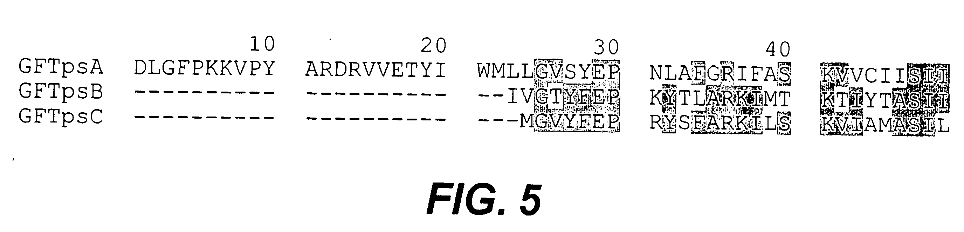

The analysis by DNA sequencing and the Blast search of the PCR products with expected length (80 to 240 bp in size) revealed fragments of three different sesquiterpene synthases, which were named GFTpsA, GFtpsB and GFTpsC. The 180-bp GFTpsA fragment was amplified with the primers TpsVF1 and TpsVR3, the 115-bp GFtpsB fragment was amplified with the primers TpsVF2 and TpsVR3, and the 115-bp GFTpsA fragment was amplified with the primers TpsVF1 and TpsVR3. The deduced amino-acid sequences revealed significant differences between these three fragments (Figure 5).

### Example 2: Isolating Sesquiterpene Synthase cDNA using 5'/3'-RACE

To isolate the full- length sequences of the sesquiterpene synthases, a 5'/3'-RACE approach was first used. cDNA was synthesized using Marathon™ cDNA Amplification Kit (Clontech) and starting from 1 µg mRNA purified from total RNA prepared with the Hot Borate technique. The quality of the synthesized cDNA was poor, essentially small size cDNAs were obtained (average size of 0.5 Kb). However, using this cDNA, the 3'-end of the GFTpsB and GFTpsC were obtained using the gene specific primers GFTpsBRF1 and GFTpsBRF2 for GFTpsB and the gene specific primers GFTpsCRF1 and GFTpsCRF2 for GFTpsC (see Table 1). mRNA prepared by the guanidinium-thiocyanate/phenol extaction method gave higher quality cDNA with an average size of 2 Kb and allowed the isolation of the 5'-end sequence of GFTpsC using the gene specific primers GFTpsCRR1 and GFTpsRR2 (see Table 1), completing the full- length sequence of this clone. The 5'-end of GFTpsB and the 5'-end and 3'-end of GFTpsA could not be obtained by this approach.

The guanidinium-thiocyanate/phenol extraction method was based on the technique described by Chomczynski and Sacchi using the RNAClean™ solution from ThermoHybaid (Chomczynski, P., and Sacchi, N., (1987) Anal. Biochem. 162, 156-159.). Briefly, 2 g of frozen tissues was crushed to a fine powder in liquid nitrogen using a mortar and pestle. The powder was transferred to 20 ml RNAClean™ solution and the suspension was homogenized using an UltraturaxTM homogenizer and filtered through MiraclothTM filter (Calbiochem). After addition of 0.1 volume of chloroform, the tube was placed on ice and centrifuged 20 min at 12000g and 4°C. The upper aqueous phase was recovered and one volume of isopropanol was added. After 20 min. incubation at -20°C, the sample was centrifuged 20 min. at 12000g and 4°C. The large white pellet obtained was washed with 70% ethanol and dried at room temperature. This pellet, containing the total RNA, was immediately subject to mRNA purification by oligodT-cellulose affinity chromatography using the FastTrack® 2.0 mRNA isolation Kit (Invitrogen). The manufacturer's protocol was followed except that after resuspension of the total RNA in the lysis buffer, the sample was heated at 100°C instead of 65°C.

3' and 5' rapid amplification of cDNA ends (RACE) were performed using Marathon™ cDNA Amplification Kit (Clontech). The procedure began with the first-strand cDNA synthesis starting from mRNA using an oligo(dT) primer. After second-strand synthesis, specific adaptors were ligated to the double stranded cDNA (ds cDNA) ends. This procedure provided an uncloned library of adaptor-ligated ds cDNA. Purified grapefruit mRNA (1 µg) was used as starting material. The quality and quantity of cDNA was evaluated on an agarose gel.

The 3'- or 5'-end of the specific cDNAs were amplified with Advantage® 2 Polymerase Mix using a combination of gene- and adaptor-specific oligonucleotides. Typical RACE reaction mixtures contained, in a final volume of 50 µl, 5 µl 10X cDNA PCR Reaction Buffer (clontech), 200 nM each dNTP, 1 µl Advantage® 2 Polymerase Mix, 200 µM adaptor-specific primer (Clontech), 200 µM gene-specific primer (see Table 1) and 5 µl of 50 to 250 fold diluted adaptor-ligated cDNA. Amplification was performed on an Eppendorf Mastercycler gradiant thermal cycler. The thermal Cycling conditions were as follows: 1 min at 94°C, 5 cycles of 30 sec at 94°C and 2 to 4 min at 72°C, 5 cycles of 30 sec at 94°C and 2 to 4 min at 70°C, 20 cycles of 30 sec at 94°C and 2 to 4 min at 68°C. When necessary a second round of amplification was performed using a nested adaptor-specific primer (Clontech) and a nested gene-specific primer.

The amplification products were evaluated, sub-cloned, and the sequence analyzed as above for the RT-PCR products.

**TABLE 1: The following primers were used in the 3'/5'-RACE experiments:**

| Name. | Description. | Sequence (5' to 3'). |
|---|---|---|
| GFTpsARF1 | 3'-RACE forward primer. | CTGGGAGTGTCCTATGAGCT CAATTTGG |
| GFTpsARF2 | GFTpsA 3'-RACE forward nested primer. | GTAGAATTTTTGCATCCAAAG TGGTGTGC |
| GFTpsARR1 | GFTpsA 5'-RACE reverse primer. | CACACCACTTTGGATGCAAA AATTCATC |
| GFTpsARR2 | GFTpsA 5'-RACE reverse nested primer. | CCAAATTGGGCTCATAGGAC ACTCCCAG |
| GFTpsBRF1 | GFTpsB 3'-RACE forward primer. | TAGGGACGTATTTTGAACCA AAGTAC |
| GFTpsBRF2 | GFTpsB 3'-RACE forward nested primer. | AAATAATGACCAAAACAATTT ACACGG |
| GFTpsBRR1 | GFTpsB 5'-RACE reverse primer. | GCACTTTCATGTATTCTGGAA G |
| GFTpsBRR2 | GFTpsB 5'-RACE reverse nested primer. | GTTTGAGCTCTTCAAAGAAA CC |
| GFTpsCRF1 | GFTpsC 3'-RACE forward primer. | AATGGGAGTGTATTTTGAGC CTCGATACTCC |
| GFTpsCRF2 | GFTpsC 3'-RACE forward nested primer. | GATATTTTCCAAAGTAATTGC AATGGCATCC |
| GFTpsCRR1 | GFTpsC 5'-RACE reverse primer. | GTTGCTAATATCCCACCTTTT |
| GFTpsCRR2 | GFTpsC 5'-RACE reverse nested primer. | AAGTGTGCCATAGGCGTCGT AGG GATAGC |
| GFTpsDRR1 | GFTpsD 5'-RACE reverse primer. | CTGTTCCGCAAGCTTAGGGG TTACATG |
| GFTpsDRR2 | GFTpsD 5'-RACE reverse nested primer. | CTGAGCTACCAATGACTTCA GGTGAGTGG |
| GFTpsERR1 | GFTpsE 5'-RACE reverse primer. | CAATTTTGCCATACACATCAT AGATATCATC |
| GFTpsERR2 | GFTpsE 5'-RACE reverse nested primer. | AACAGAAGTCATGGAGATCA CTTTCGTC |
| GFTpsERR3 | GFTpsE 5'-RACE reverse primer. | CGCAAGAGATGTTTTAAAGTT CCCATCC |
| GFTpsERR4 | GFTpsE 5'-RACE reverse nested primer. | TGAACATCAGCGGAAATTTTA TAGCC |

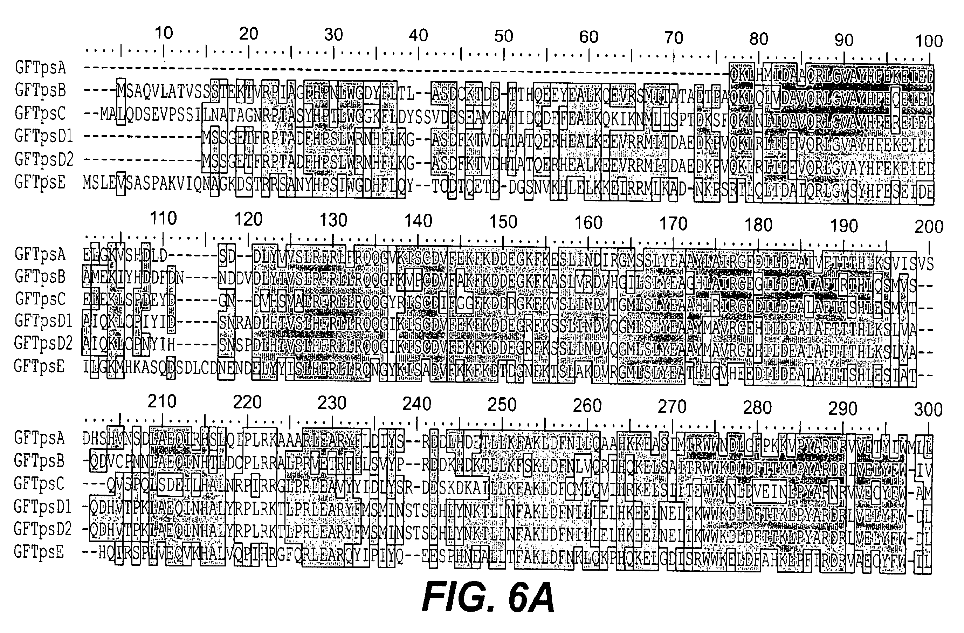

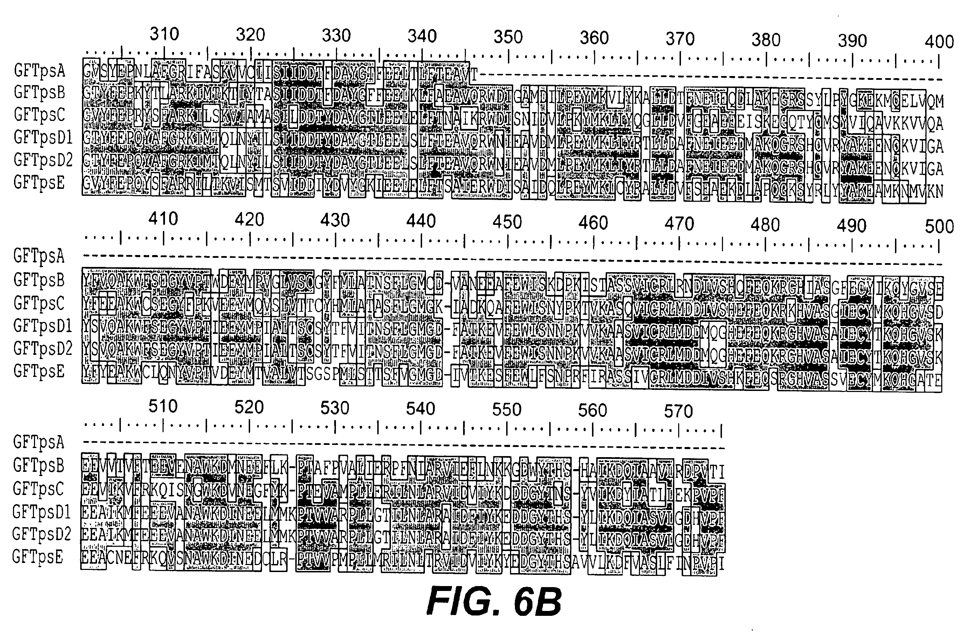

The partial GFTpsB cDNA obtained by 3'-RACE revealed high sequence identity to a putative terpene synthase cDNA found in the public databases (GeneBank accession no. AF288465) and isolated from Citrus junos. Primers specific for the sequence of this terpene synthase were designed: one pair of forward and reverse primers (junosF1 and junosR1) designed against the non-coding region of the C. junos terpene synthase and one pair of forward and reverse primers (junosF2 and junos R2) designed in the coding region including the start and stop codons. PCR on the grapefruit Marathon™ cDNA library using the primers junosF1 and junosR1 produced no amplicon. Using the junosF2 and junosR2 primers a fragment of 1.6 Kb was amplified. DNA sequencing confirmed this PCR product as being a 1669-bp full-length sesquiterpene synthase of the GFTpsB clone. (Figure 6).

### Example 3: cDNA library screening and EST sequencing

In order to obtain the full-length cDNA of the partial clones obtained by the PCR approach, a cDNA library prepared from grapefruit flavedo mRNA was constructed.

cDNA synthesis and library construction were performed using the Uni-ZAP® XR library construction Kit (Stratagene) according to manufacturer's protocol starting from 7.5 µg grapefruit flavedo mRNA (prapared by the Guanidinium-Thiocyanate/Phenol method). The original titer of the library was 2x10⁷ PFU (plaques forming units) and the average insert size was 1.1 Kb.

Two approaches were used to isolate sesquiterpene synthases encoding cDNAs from the cDNA library : EST sequencing and screening using a DNA probe. For EST (expressed sequence tag) sequencing, a fraction of the library was used to excise the pBluescript phagemid from the Uni-ZAP XR vector according to Stratagene's mass excision protocol. Resulting transformed bacterial colonies (576) were randomly picked and the plasmid purified. One sequencing reaction was performed for each clone using the T3 primer. The sequences were first edited to remove vector sequences and compared against the GenBank non redundant protein database (NCBI) using the BLASTX algorithm (Altschul et al 1990).

The library was screened with digoxigenin (DIG)-labeled DNA probes. The probes were synthesized by incorporation of DIG-dUTP into a DNA fragment by PCR using the PCR DIG (digoxigenin) Probe Synthesis Kit (Roche Diagnostics). A 149-bp GFTpsA-derived DNA probe was amplified from an aliquot of the library using the forward primer GFTpsAproF (5'-ACGATTTAGGCTTCCCTAAAAAGG-3') and the reverse primer GFTpsAproR (5'-TATTATGGAATATTATGCACACCAC-3'). A 1036-bp GFTpsB-derived probe was amplified from the pET-GFTpsB2-2 plasmid using the forward primer junosF2 (5'-AAATGTCCGCTCAAGTTCTAGCAACGG-3') and the reverse primer GFTpsBRR2 (5'-GTTTGAGCTCTTCAAAGAAACC-3'). A 1008-bp GFTpsC-derived DNA probe was amplified from the pET-GFTpsC plasmid using the forward primer GFTpsCpetF1 (5'-ATGGCACTTCAAGATTCAGAAGTTCC-3') and the reverse primer GFTpsCRR1 (5'-GTTGCTAATATCCCACCTTTTGATAGC-3').

The probes were hybridized to plaques lifts, prepared from plates containing 5,000 to 25,000 PFU, at 40°C in Dig Easy Hyb hybridization solution (Roche Diagnostics). The detection of the probe-target hybrids on the membranes was performed by chemiluminescence using Anti-Digoxigenin-Alkaline Phosphatase (Roche Diagnostics) and CDP-Star alkaline phosphate substrate (Roche Diagnostics), and the visualization was made on a VersaDoc Imaging System (BioRad).

Phages from positives signals were cored from the agar plates and subject to secondary and if necessary to tertiary screening to achieve single plaques positive signals. The positive isolates were in vivo excised and the insert sequenced as described above.

The library was screened using DNA probes prepared from GFTpsA, GFTpsB and GFTpsC. This approach yielded three different sesquiterpene synthase cDNAs. Two of them were previously found clones: one clone, named GF2-30-1, was a 5'-end 300-bp truncated form of the GFTpsC cDNA; the second clone, named 9-13-6, was a partial clone of GFtpsA that was truncated at its 5'-end by approximately 168-bp as judged by comparison to other sesquiterpene synthases. The 9-13-6 clone provided 618-bp additional 5'-end sequence information compared to the above mentioned partial GFTpsA clone obtained by RT-PCR. At its 3'-end, the 9-13-6 clone was also incomplete. Approximately 680 nucleotides were missing and were replaced by a 600-bp fragment of no defined function. The third sesquiterpene synthase encoding cDNA obtained by screening, GF2-5-11, encoded a new sesquiterpene synthase that was named GFTpsD. This clone was truncated at the 5'-end, but the missing 414-bp was recovered by 5'-RACE using the primers described in Table 1. The full-length GFTpsD was then amplified from Marathon™ cDNA library and cloned in the bacterial expression vector as described above. Analysis of the DNA sequence of several full-length GFTpsD cDNAs revealed that two closely related sesquiterpene synthases with minor sequence differences were present, they were named GFTpsD1 and GFTpsD2 (Figure 6). The deduced amino-acid sequence of these two variants differed from each other by 4 residues.

For the EST sequencing approach, 576 clones were sequenced. Among them, only three clones encoded putative terpene-synthases like proteins and more precisely, two different sesquiterpene synthase-like and one monoterpene synthase-like proteins. One of the two sesquiterpene synthase-like clones (clone GF002-G3) was the previously identified clone GFTpsD1. The second (clone GF006-G7) was a truncated cDNA encoding a new sesquiterpene synthase that was named GFTpsE. This clone was again 5'-end truncated (by approximately 800 bp) and the missing fragment could be amplified by 5'-RACE in two stages as follows. A first 5'-RACE using the primers GFTpsERR1 and GFTpsERR2 (see Table 1) provided 500 additional 5'-end nucleotides and a second 5'RACE using the primers GFTpsERR3 and GFTpsERR4 (see Table 1) provided the missing 5'-end DNA sequence to reconstitute the full-length GFTpsE cDNA (Figure 6).

### Example 4: Construction of Plasmids and Enzyme expression

### Construction of expression plasmids.

The cDNA were sub-cloned in the pET11a expression plasmid (Novagen) for functional expression of the sesquiterpene synthases. For GFTpsB, GFtpsD and GFTpsE, the full-length cDNAs were amplified by PCR to introduce an NdeI site at the 5'-end including the start codon and a BamHI site at the 3'-end immediately after the stop codon. For GFTpsB, the forward primer GFTpsBNdeI 5'-GCATGTTCC*A*T*ATG*TCCGCTCAAGTTCTAGCAACGGTTTCC-3' (Ndel site in italics, stat codon underlined) and the reverse primer GFTpsBBam 5'-CGCGG*AT*CCTCAGATGGTAACAGGGTCTCTGAGCACTGC-3' (BamHI site in italics, stop codon underlined) were used. In the same way, the forward primer GFTpsDNdeI 5'-GCATGTTCC*ATATG*TCGTCTGGAGAAACATTTCGTCC-3' and the reverse primer GFTpsDBam 5'-CGC*GGATCC*TCAAAATGGAACGTGGTCTCCTAG-3' were used for GFTpsD and the forward primer GFTpsENdeI 5'-GCATGTTC*CAT**ATG***TCTTTGGAAGTTTCAGCCTCTCCTG-3' and the reverse primer GFTpsEBam 5'-CGCGG*ATCC*TCATATCGGCACAGGATTAATAAACAAAGAAGC-3' were used for GFTpsE.

The amplifications were performed using the Pfu DNA polymerase (Promega) in a final volume of 50 µl containing 5µl of Pfu DNA polymerase 10X buffer, 200 µM each dNTP, 0.4 µM each forward and reverse primer, 2.9 units Pfu DNA polymerase and 5 µl of 100-fold diluted cDNA (prepared as described above using the Marathon™ cDNA Amplification Kit (Clontech)). The thermal cycling conditions were as follows: 2 min at 95°C; 25 cycles of 30 sec at 95°C, 30 sec at 55°C and 4 min at 72°C; and 10 min at 72°C. The PCR product was purified on an agarose gel, eluted using the QIAquick® Gel Extraction Kit (Qiagen), digested with NdeI and BamHI and ligated into the similarly digested pET11 a plasmid.

For construction of the GFTpsC-pET11 a expression vector, two separated PCR were employed to generate the insert flanked with the Ndel and BamHI cohesive ends. A first PCR was performed in the same condition as described above, using the forward primer GFTpsCpETF1 5'-TAATGGCACTTCAAGATTCAGAAGTTCCTC-3' and the reverse primer GFTpsCpETR1 5'-AAAAGGGAACAGGCTTCTCAAGCAATG-3' and a second PCR was performed using the forward primer GFTpsCpETF2 (shortened by AT at the 5'-end compared to GFTpsCpETF1) 5'-ATGGCACTTCAAGATTCAGAAGTTCCTC-3' and the reverse primer GFTpsCpETR2 (extended by GATC at the 5'-end compared to GFTpsCpETR1) 5'-GATCAAAAGGGAACAGGCTTCTCAAGCAATG-3'. The two PCR products were purified as described above, combined, denatured by 5 min boiling and cooled 5 min on ice. The resulting cDNA was used directly for ligation into the pET11 a plasmid.

Ligation products were initially transformed into JM109 E. coli cells and the constructs were verified by restriction digestion and DNA sequencing.

### Sesquiterpene synthases expression.

For protein expression, the pET11 a plasmids containing the sesquiterpene synthase cDNAs as well as the empty pET11a plasmid were transformed into the BL21 (DE3) E. coli cells (Novagen). Single colonies were used to inoculate 5 ml LB medium. After 5 to 6 hours incubation at 37°C, the cultures were transferred to a 20°C incubator and left 1 h for equilibration. Expression of the protein was then induced by addition of 0.5 mM IPTG and the culture incubated over-night at 20°C.

The next day, the cells were collected by centrifugation, resuspended in 0.5 ml Extraction Buffer (50 mM MOPSO pH 7, 5 mM EDTA, 5 mM EDTA, 10% glycerol) and sonicated 3 times 30 s. The cell debris were sedimented by centrifugation 30 min at 18,000g and the supernatant containing the soluble proteins was recovered. The expression of the sesquiterpene synthases was evaluated by separation of the protein extract on a SDS-PAGE (SDS-polyacrylamid gel electrophoresis) and staining with coomassie blue and comparison to protein extract obtained from cells transformed with the empty plasmid.

A distinct band with the expected calculated molecular weight was observed for all constructs and the band was not present in the soluble proteins from *E. coli* transformed with the empty plasmid.

### Example 5 Enzyme Function Assay

The enzymatic assays were performed in sealed glass tubes using 50 to 100 µl of protein extract in a final volume of Extraction Buffer supplemented with 15 mM MgCl₂ and 100 to 250 µM FPP (Sigma). The medium were overlaid with 1 ml pentane and the tubes incubated over-night at 30°C. The pentane phase, containing the sesquiterpenes, was recovered and the medium extract with a second volume of pentane. The combined pentane fractions were concentrated under nitrogen and analyzed by Gas Chromatography on a on a Hewlett-Packard 6890 Series GC system using a 0.25 mm inner diameter by 30 m SPB-1 (Supelco) capillary column. The carrier gas was He at constant flow of 1.6 ml/min. Injection was done at a split ratio of 2:1 with the injector temperature set at 200°C and the oven programmed from 80°C (0 min hold) at 7.5°C/min to 200°C (0 min hold) followed by 20°C/min to 280°C (2 min hold). Detection was made with a flame ionization detector. Compound identification was based on retention time identity with authentic standards when available. For confirmation of the products identities, samples were analyzed by combined capillary GC-MS using a Hewlett-Packard 6890 GC-quadrupole mass selective detector system, equipped with a 0.25 mm inner diameter by 30 m SPB-1 (Supelco) capillary column. The oven was programmed from 80°C (0 min hold) to 280°C at 7.5°C at a constant flow of 1.5 ml/min He. The spectra were recorded at 70eV with an electron multiplier voltage of 2200V.

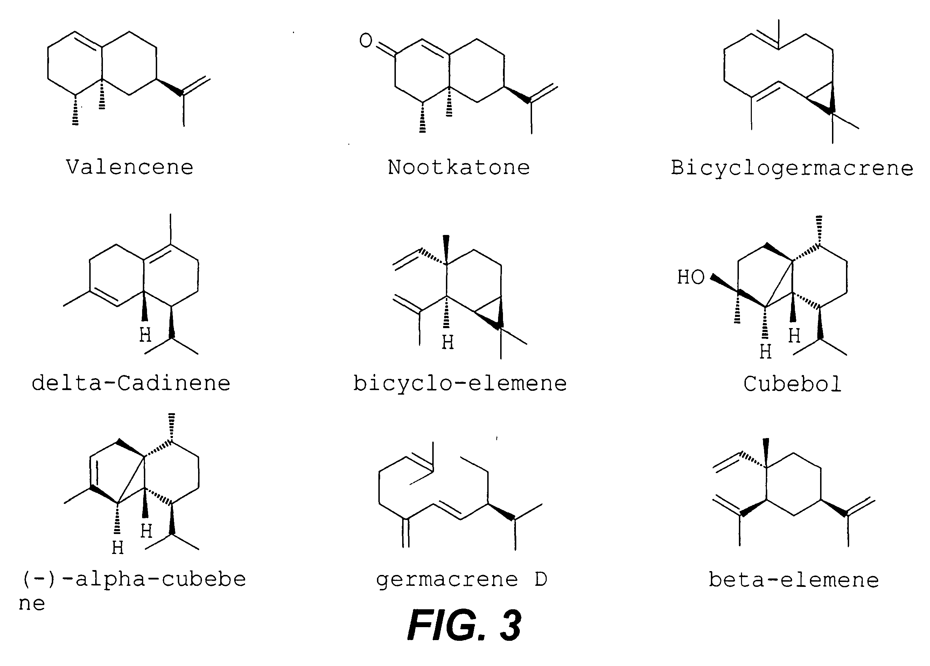

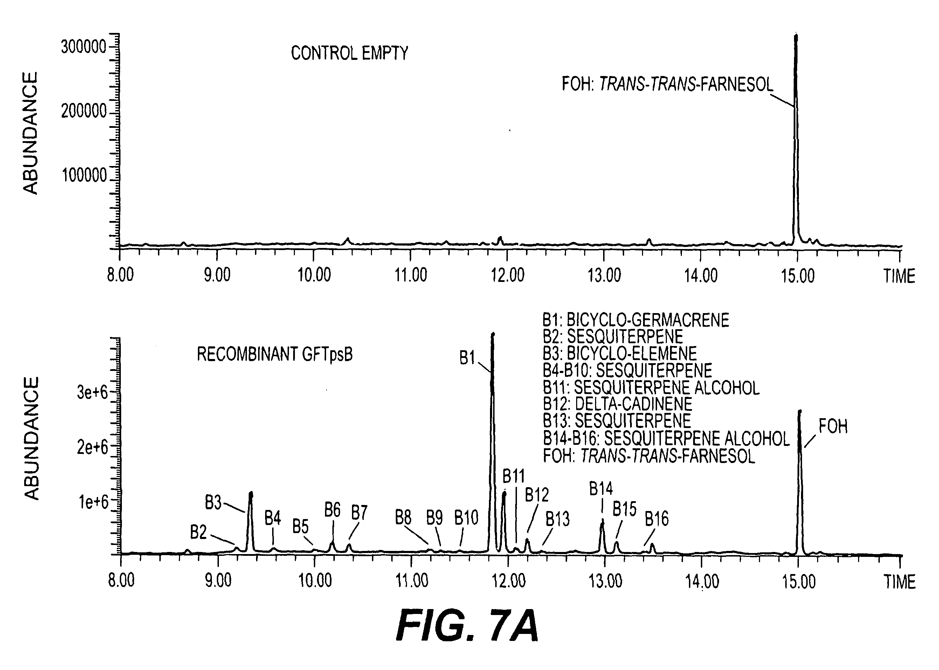

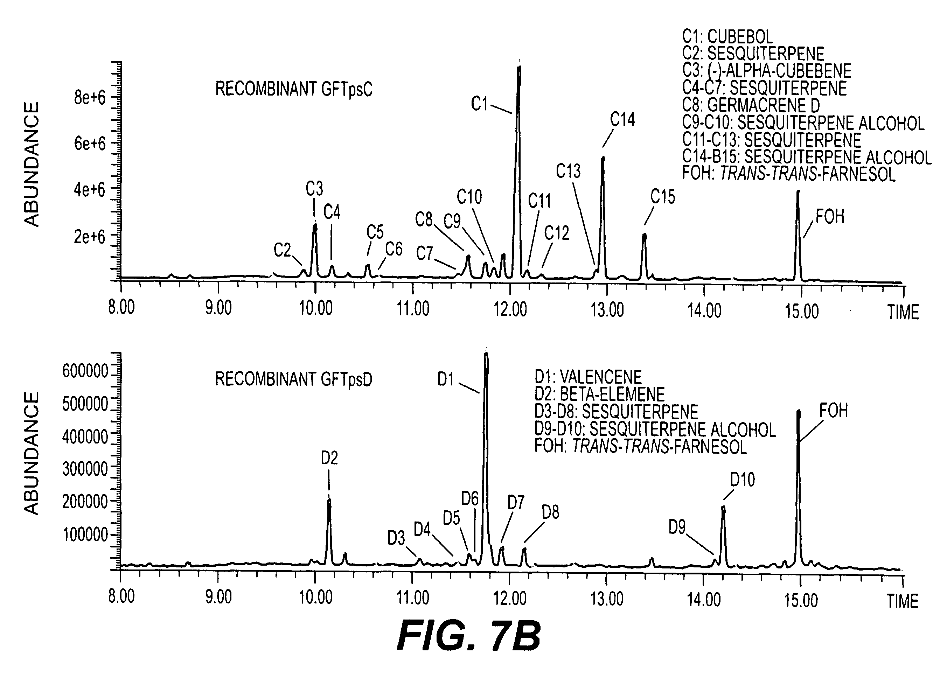

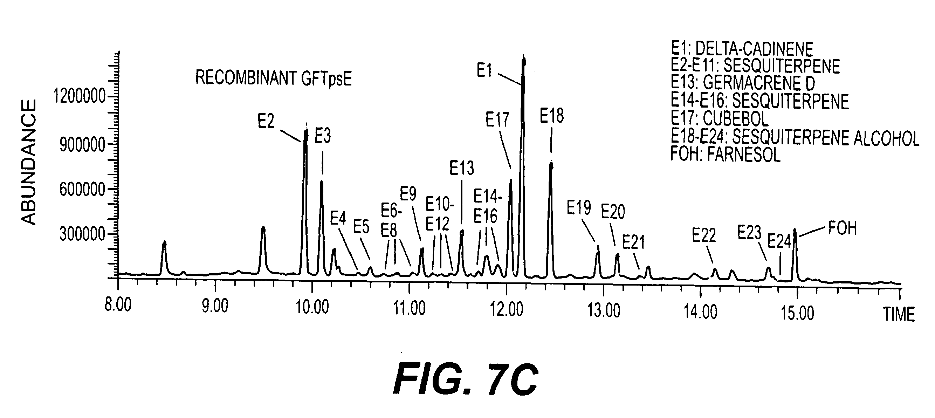

The enzyme activity of the different recombinant enzymes were evaluated in this assay using the soluble protein fraction and farnesyl-diphosphate as substrate. Sesquiterpene synthase activity was obtained for all clones tested and the product formed were characterized by retention time and GC-MS (Figure 7). The GFTpsB cDNA encoded a sesquiterpene synthase producing bicyclo-germacrene (Figure 3) as major product and at least 15 minor sesquiterpene olefins or oxygenated sesquiterpenes such as delta-cadinene (Figure 3) (byclo-elemene (Figure 3), resulting from heat rearrangement of bicyclo-germacrene was also observed in the GC trace). The GFTpsC cDNA encoded a multiple product forming sesquiterpene synthase with a major product being identified as cubebol (Figure 3). The enzyme also produced 3 other sesquiterpenes in a relative large proportion (-)-α-cubebene and 2 oxygenated sesquiterpenes, and in small amounts, at least 11 sesquiterpene olefins olefins or oxygenated sesquiterpenes. GFtpsD encoded a sesquiterpene synthase that produces Valencene as a major product. Ten other minor peaks were also identified as sesquiterpene olefins or alcohols. Among them, β-elemene is a heat degradation product of germacrene A. GFTpsE encoded a sesquiterpene synthase producing a complex mixture of sesquiterpene compounds with δ-cadinene (figure 3) being slightly the most abundant. Cubebol and Germacrene D (Figure 3) were also identified in the mixture of products formed by GFTpsE.

The isolated sesquiterpene synthases demonstrated multiple product forming properties in the experiments described. For example, the cubebol synthase and the δ-cadinene produce 3 or 5 products in relatively large proportion. The bicyclogermacrene synthase produced a major sesquiterpene and several secondary products in trace amounts.

## Claims

1. A method of making valencene comprising
A) contacting farnesyl pyrophosphate with at least one valencene synthase encoded by a nucleic acid chosen from
(a) a nucleic acid comprising the nucleotide sequence of Figure 8(d) or 8(e); or
(b) a nucleic acid encoding the polypeptide of Figure 8(d) or 8(e); and
B) isolating the valencene produced in A).

2. The method of claim 1, wherein the pH at the time of contacting is 7 or less.

3. The method of claim 1 or 2, wherein the at least one valencene synthase is produced by culturing a host cell comprising the nucleic acid, wherein the host cell is chosen from bacterial cells, yeast cells, plant cells, and animal cells.

4. The method of claim 3, wherein the host cell is a microbial cell, preferably *E. coli.*

5. A host cell comprising a nucleic acid chosen from
(a) a nucleic acid comprising the nucleotide sequence of Figure 8(d) or 8(e); or
(b) a nucleic acid encoding the polypeptide of Figure 8(d) or 8(e).

6. The host cell of claim 5, wherein said host cell is a prokaryote, a yeast or a higher eukaryotic cell.

7. The host cell of claim 6, wherein said prokaryote is E. Coli and wherein said yeast is *Saccharomyces cerevisiae.*

8. A plant or a microorganism modified to harbor a nucleic acid chosen from
(a) a nucleic acid comprising the nucleotide sequence of Figure 8(d) or 8(e); or
(b) a nucleic acid encoding the polypeptide of Figure 8(d) or 8(e).

## Patentansprüche

1. Verfahren zum Herstellen von Valencen, umfassend
(A) das Kontaktieren von Famesylpyrophosphat mit mindestens einer Valencensynthase, die durch eine Nucleinsäure kodiert wird, die ausgewählt wird unter
(a) einer Nucleinsäure, die eine Nucleotidsequenz aus Figur 8(d) oder 8(e) umfasst; oder
(b) einer Nucleinsäure, die für das Polypeptide der Figur 8(d) oder 8(e) kodiert ; und
(B) das Isolieren des in (A) hergestellten Valencens.

2. Verfahren nach Anspruch 1, wobei der pH-Wert zum Zeitpunkt des Kontaktierens 7 oder weniger beträgt.

3. Verfahren nach Anspruch 1 oder 2, wobei die mindestens eine Valencensynthase durch Züchten einer Wirtzelle, die Nucleinsäure umfasst, hergestellt wird, wobei die Wirtzelle unter Bakterienzellen, Hefezellen, Pflanzenzellen und Tierzellen ausgewählt wird.

4. Verfahren nach Anspruch 3, wobei die Wirtzelle eine mikrobielle Zelle, bevorzugt *E. coli,* ist.

5. Wirtszelle umfassend eine Nucleinsäure ausgewählt unter
(a) einer Nucleinsäure, die eine Nucleotidsequenz aus Figur 8(d) oder 8(e) umfasst; oder
(b) einer Nucleinsäure, die für das Polypeptide der Figur 8(d) oder 8(e) kodiert.

6. Wirtszelle nach Anspruch 5, wobei die Wirtszelle ein Prokaryot, eine Hefe oder eine höhere eukaryotische Zelle ist.

7. Wirtszelle nach Anspruch 6, wobei der Prokaryot E. coli ist und wobei die Hefe *Saccharomyces cerevisiae* ist.

8. Pflanze oder Mikroorganismus, die/der zum Beherbergen einer Nucleinsäure modifiziert ist, die ausgewählt wird unter
(a) einer Nucleinsäure, die eine Nucleotidsequenz aus Figur 8(d) oder 8(e) umfasst; oder
(b) einer Nucleinsäure, die für das Polypeptide der Figur 8(d) oder 8(e) kodiert.

## Revendications

1. Procédé de préparation de valencène comprenant
A) la mise en contact de farnésyl-pyrophosphate avec au moins une valencène synthase codée par un acide nucléique choisi parmi
(a) un acide nucléique comprenant la séquence nucléotidique de la figure 8(d) ou 8(e); ou
(b) un acide nucléique codant pour le polypeptide de la figure 8(d) ou 8(e); et
B) l'isolement du valencène produit en A).

2. Procédé selon la revendication 1, dans lequel le pH au moment de la mise en contact est inférieur ou égal à 7.

3. Procédé selon la revendication 1 ou 2, dans lequel ladite au moins une valencène synthase est produite par culture d'une cellule hôte comprenant l'acide nucléique, la cellule hôte étant choisie parmi les cellules bactériennes, les cellules de levures, les cellules végétales, et les cellules animales.

4. Procédé selon la revendication 3, dans lequel la cellule hôte est une cellule microbienne, de préférence *E. coli.*

5. Cellule hôte comprenant un acide nucléique choisi parmi
(a) un acide nucléique comprenant la séquence nucléotidique de la figure 8(d) ou 8(e); ou
(b) un acide nucléique codant pour le polypeptide de la figure 8(d) ou 8(e).

6. Cellule hôte selon la revendication 5, ladite cellule hôte étant un procaryote, une levure ou une cellule d'eucaryote supérieur.

7. Cellule hôte selon la revendication 6, ledit procaryote étant *E. coli* et ladite levure étant *Saccharomyces cerevisiae.*

8. Plante ou micro-organisme modifié(e) pour héberger un acide nucléique choisi parmi
(a) un acide nucléique comprenant la séquence nucléotidique de la figure 8(d) ou 8(e); ou
(b) un acide nucléique codant pour le polypeptide de la figure 8(d) ou 8(e).
